# EUROPEAN PATENT APPLICATION

(11) **EP 0 994 107 A1**
(43) Date of publication of application: **19.04.2000**
(21) Application number: 99125828.6
(22) Date of filing: 02.11.1994
(51) Int. Cl.: C07D 261/12, A61K 31/42, C07D 413/04, C07D 417/04, C07D 413/14

(54) **5-Arylisoxazol-4-yl-substituted 2-amino carboxylic acid compounds**

(30) Priority: 03.11.1993 DK 124393
(62) Divisional of application: 94931523.8
(71) Applicant: H. LUNDBECK A/S, 2500 Copenhagen (DK)
(72) Inventor: The designation of the inventor has not yet been filed
(74) Representative: Meidahl Petersen, John

(57) **Abstract**

2-Aminocarboxylic acid compounds substituted with 5-arylisoxazol-4-yl or 5-arylisothiazol-4-yl and having general formula (I), wherein A is a bond or a spacer group; B is a group -CH(NR'R'')-COOH wherein R' and R'' are independently hydrogen or C₁₋₆ alkyl, or B is a group of formula (II), wherein R₂, R₃ and R₄ are substituents; or R₃ and R₄ or R₄ and R₂ are connected in order to form a ring; E is O, S, COO, (CH₂)ₙ-COO, O-(CH₂)ₙ-COO or S-(CH₂)ₙ-COO wherein n is 1-6, 5-tetrazolyl, 5-tetrazolyl-C₁₋₆ alkyl, 3-hydroxyisoxazolyl or 3-hydroxyisoxazolyl-C₁₋₆ alkyl; D is O or S; and R₁ is an optionally substituted aryl or heteroaryl group; are excitatory amino acid receptor ligands useful in the treatment of cerebral ischaemia, Huntington's disease, epileptic disorders, Parkinson's disease, Alzheimer's disease, schizophrenia, pain, depression and anxiety.

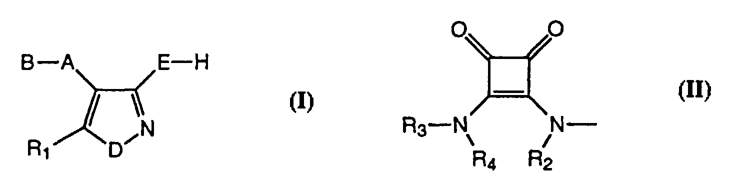

## Description

### Field of the Invention.

The present invention relates to a novel class of (5-arylisoxazol-4-yl)-substituted 2-amino carboxylic acid derivatives, which are excitatory amino acid (EAA) receptor ligands useful in the treatment of cerebral ischaemia, Huntington's disease, epileptic disorders, Parkinson's disease, Alzheimer's disease, schizophrenia, pain, depression and anxiety.

### Background of the Invention.

As a result of extensive studies of excitatory mechanisms in the central nervous system (CNS) during the past three decades, there is now a consensus of opinion that *(S)*-glutamate (Glu) is the major EAA neurotransmitter in the CNS (Lodge, D. *Excitatory Amino Acids in Health and Disease*. J. Wiley & Sons: Chichester, **1988**; Wheal, H.; Thomson, A. *Excitatory Amino Acids and Synaptic Transmission*. *Academic Press*: London, **1991**; Meldrum, B.S. *Excitatory Amino Acid Antagonists*. *Blackwell Sci. Publ*.*:* Oxford, **1991**; Krogsgaard-Larsen, P.; Hansen, J.J. *Excitatory Amino Acid Receptors: Design of Agonist and Antagonists*. E. Horwood: *Chichester*, **1992**). Glu-operated neurotransmission is mediated by a large number of receptors, classified into at least five heterogeneous families of receptors named NMDA, AMPA, kainic acid, metabotropic, and L-AP4 classes of receptors (Monaghan, D.T., et al. *Ann. Rev. Pharmacol*. *Toxicol*. **1989**,*29*, 365-402; Watkins, J.C.; Krogsgaard-Larsen, P.; Honoré, T. *Trends Pharmacol Sci*. **1990**,*11*, 25-33; Simon, R.P. *Excitatory Amino Acids*. *Thieme Med. Publ*.: New York, **1992**).

There is very strong evidence supporting the view that excessive excitation mediated by EAA receptors ("excitotoxicity") is a factor of major importance in cerebral ischaemia following stroke, head injury, asphyxia, subarachnoid haemorrhage, cardiac arrest and other situations (Lodge, D., **1988** *supra*; Meldrum, B.S., **1991** *supra*). It has been shown in animal models that the damages caused by various ischaemic conditions can be inhibited by the administration of Glu-antagonists. So, although the relative importance of the different classes of EAA receptors in the phenomena underlying ischaemic insults is unclear, it is generally agreed that EAA receptor antagonists are potential therapeutic agents in these conditions.

Accumulating evidence derived from different lines of neurochemical and pharmacological research suggests that derailed EAA receptor mechanisms, possibly including "excitotoxicity", play a role in Huntington's disease (Young, A.B.; et al. *Science* **1988**,*241*, 981-983), epileptic disorders (Krogsgaard-Larsen, P.; Hansen, J.J., **1992** *supra*), Parkinson's disease (Klockgether, T.; Turski, L. *Trends. Neurosci*. **1989**,*12*, 285-286), and Alzheimer's disease (Greenamyre, J.T.; Maragos, W.F. *Cerebrovasc*. *Brain. Metab. Rev.* **1993**,*5*, 61-94; Francis, P.T., et al. *J. Neurochem.* **1993**,*60*,1589-1604).

Furthermore, central EAA receptors may be involved in the synaptic mechanisms underlying schizophrenia (Reynolds, G.P. *Trends. Pharmacol. Sci.* **1992**,*13*,116-121), pain and anxiety (Drejer, J. In: *Excitatory Amino Acid Receptors: Design of Agonists and Antagonists* (Eds. Krogsgaard-Larsen, P.; Hansen, J.J.) E. Horwood: Chichester **1992**, pp. 352-375) and depression (Trullas, R., Skolnick, P., *Eur. J. Pharmacol.* **1990**, *185*, 1-10 and Trullas et al., *Eur. J. Pharmacol.* **1991**, *203*, 379-385. So, reduced function of EAA receptors (EAA hypoactivity) seems to play a role in, for example, schizophrenia (Deutsch, S.I.; et al. *Clin. Neuropharmacol.* **1989**,*12*, 1-13) and some of the clinical symptoms seen in Alzheimer's disease (Greenamyre, J.T.; et al. Prog. *Neuro-Psychopharmacol. & Biol. Psychiat.* **1988**,*12*, 421-430 ). It is possible that "excitoxicity" as well as EAA hypoactivity are involved in the complex mechanisms associated with Alzheimer's disease (Greenamyre, J.T.; **1988** *supra*; Greenamyre, J.T.; Maragos, W.F., **1993**, *supra*).

Accordingly, EEA receptor ligands are considered to be useful in the treatment of cerebral ischaemia, Huntington's disease, epileptic disorders, Parkinson's disease, Alzheimer's disease, anxiety, schizophrenia, depression and pain.

Most EAA receptor agonists so far tested, show more or less pronounced neurotoxicity in model systems and consequently clinical uses of such compounds may be limited (Carlsson, M.; Carlsson, A. *Trends. Neurosci.* **1990**,*13*, 272-276) (Willetts, J.; Balster, R.L.; Leander, J.D. *Trends. Pharmacol. Sci.* **1990**,11,423-428).

Partial EAA agonists showing appropriate balance between agonism and antagonism, may on the other hand, have considerable therapeutic interest, cf. the above indications, (Greenamyre, J.T.; **1988** *supra*;; Christensen, I.T.; et al. *Drug. Des. Del*. **1989**,*5*, 57-71; Francis, P.T.; et al. *J. Neurochem.* **1993**,*60*, 1589-1604). Partial agonists may, by virtue of their EAA antagonist profile, show therapeutically useful neuroprotection and, at the same time, be sufficiently agonistic to prevent total blockade of the neurotransmission mediated by the particular EAA receptor.

ATPA, the 5-*tert*-butyl analogue of AMPA ((*RS*)-2-amino-3-(3-hydroxy-5-methylis-oxazol-4-yl)propionic acid), has been disclosed to be systemically active whereas it has not been reported to show neurotoxic effects in animals (Ornstein, P.L.; et al. *J. Med. Chem.* **1993**,*36*, 2046-2048; Lauridsen, J.; Honoré, T.; Krogsgaard-Larsen, P. *J. Med. Chem.* **1985**, *28*, 668-672).

Like AMPA itself, a number of mono- and bicyclic AMPA analogues have been found to show selective agonist effects at AMPA receptors (Hansen, J.J.; Krogsgaard-Larsen,P. *Med. Res. Rev.* **1990**,*10*, 55-94; Krogsgaard-Larsen, P.; Hansen, J.J., **1992** *supra*;). One of these analogues, (*RS*)-2-Amino-3-(3-hydroxy-5-phenylis-oxazol-4-yl)propionic acid (APPA), in which the methyl group of AMPA has been replaced by a phenyl group, shows a weak but unique partial agonist profile (Christensen, I.T.; et al.,**1989**,*supra*).

As seen from the above evidence non-neurotoxic, CNS-active EEA receptor ligands with good penetration into the CNS are highly desirable for treating the various diseases mentioned and, accordingly, it is the object of the present invention to provide such new drugs.

### Summary of the invention

It has now been found that a novel class of (5-arylisoxazol-4-yl)-substituted 2-amino carboxylic acid derivatives are potent EAA receptor ligands.

Accordingly, the present invention relates to a novel class of (5-arylisoxazol-4-yl)-or (5-arylisothiazol-4-yl)-substituted 2-amino carboxylic acid compounds having general Formula I 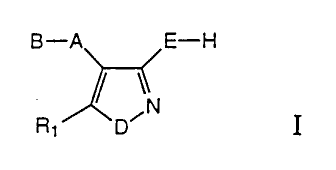
wherein A is a bond or a spacer group selected from C₁₋₆ alkylene, C₂₋₆ alkenylene or C₂₋₆ alkynylene, and cycloalkylene;
B is selected from a group -CH(NR'R'')-COOH wherein R' and R'' are independently hydrogen or C₁₋₆ alkyl, and a group of Formula II 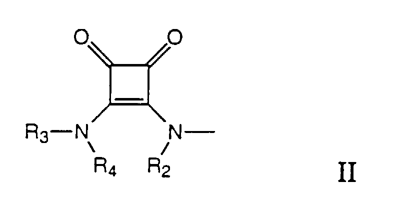
wherein R₂, R₃ and R₄ are independently selected from the group consisting of
   a) hydrogen, C₁₋₆ alkyl, C₂₋₆ alkenyl, C₂₋₆ alkynyl, cycloalk(en)yl, cycloalk(en)yl-C₁₋₆ alk(en/yn)yl, phenyl-C₁₋₆ alkyl, thienyl-C₁₋₆-alkyl, and
   b) C₁₋₆ alkyl, C₂₋₆ alkenyl and C₂₋₆ alkynyl in which one or more carbon atoms are replaced by N, O, and/or S; or
R₃ and R₄ are connected thereby forming a C₂-C₆ alkylene, C₂-C₆ alkenylene or C₂-C₆ alkynylene group; or
R₄ and R₂ are connected in order to form a C₁-C₃ alkylene, C₂-C₃ alkenylene or C₂-C₃ alkynylene group optionally mono- or di-substituted with hydroxy or methyl, or CH₂-O-CH₂;
E is O, S, COO, (CH₂)ₙ-COO, O-(CH₂)ₙ-COO, or S-(CH₂)ₙ-COO in which groups n is an integer of 1-6, a 5-tetrazolyl group, a 5-tetrazolyl-C₁₋₆ alkyl group, a 3-hydroxyisoxazolyl group or a 3-hydroxyisoxazolyl-C₁₋₆ alkyl group;
D is O or S; and
R₁ is an aryl or heteroaryl group or an aryl or heteroaryl group substituted with one or more substituents selected from halogen, C₁₋₆ alkyl, C₁₋₆ alkoxy, hydroxy, C₁₋₆ alkylthio, C₁₋₆ alkylsulfonyl, C₁₋₆ alkylamino or di-(C₁₋₆ alkyl)amino, cyano, nitro, trifluoromethyl, or trifluoromethylthio;
   provided that when A is methylene, B is a group -CH(NH₂)-COOH, E is O, D is O, and R₁ is phenyl or phenyl substituted with halogen or methoxy; then the compound must be in an enantiomeric pure form.

In another aspect the invention relates to a method for the preparation of the novel compounds of Formula I.

In yet another aspect the invention relates to a pharmaceutical composition comprising a novel compound of Formula I together with a suitable pharmaceutically acceptable carrier or diluent.

In yet another aspect the invention relates to the use of a compound of Formula I for preparing a pharmaceutical composition for treatment of cerebral ischaemia, Huntington's disease, epileptic disorders, Parkinson's disease, Alzheimer's disease, schizophrenia, pain, depression or anxiety.

Some of the compounds of the invention have been found to be selective AMPA receptor ligands *in vitro* with affinities in the low micromolar concentrations whereas other compounds have been found selectively to bind to NMDA receptors *in vitro*. Other compounds of the invention have been found to show affinity to both AMPA and NMDA receptors, respectively, *in vitro*. Furthermore, some of the compounds of the invention were found to be agonists whereas others were found to be antagonists. Thus the compounds of the invention are useful in the treatment of cerebral ischaemia, Huntington's disease, epileptic disorders, Parkinson's disease, Alzheimer's disease, schizophrenia, pain, depression and anxiety.

### Detailed Description of the invention

Some of the compounds of general Formula I may exist as optical isomers thereof and such optical isomers are also embraced by the invention.

In general Formula I, the term C₁₋₆ alkyl is intended to mean a straight chain or branched alkyl group having from 1 to 6 C atoms, inclusive, such as methyl, ethyl, 1 -propyl, 2-propyl, 1-butyl, 2-butyl, 2-methyl-2-propyl etc. Similarly, C₂₋₆ alkenyl and C₂₋₆ alkynyl designate such straight chain or branched groups having 2 to 6 C-atoms and C₁₋₆ alkylene, C₂₋₆ alkenylene and C₂₋₆ alkynylene designate such branched or straight chain divalent groups. Cycloalkyl designates such a group having 3-7 carbon atoms.

The term "alk(en/yn)yl" means that the group may be an alkyl, alkenyl or alkynyl group.

The term bond (defined for A) means that B may be attached directly to the 4-position of the isoxazole ring.

Halogen means fluoro, chloro, bromo or iodo.

The term aryl is intended to mean a carbocyclic aromatic monocyclic or fused bicyclic group or a biphenyl group and the term heteroaryl is intended to mean an aromatic monocyclic or bicyclic group containing at least one heteroatom. Examples of such groups are 5-membered aromatic heteroaryl groups comprising 1-4 heteroatoms selected from N, O and S such as thienyl, furyl, pyrolyl, oxazolyl, isoxazolyl, thiazolyl, isothiazolyl, pyrazolyl, imidazolyl, triazolyl, oxadiazolyl, thiadiazolyl and tetrazolyl. Further examples are benzothienyl, benzofuranyl, indolyl, phenyl, pyridyl, pyrimidinyl, pyridazinyl, pyrazinyl, naphthyl, quinolyl, quinazolinyl, quinoxalinyl and cinnolinyl.

Some of the compounds of the general Formula I may exist as pharmaceutically acceptable salts thereof which are also embraced by the invention.

The salts of the compounds of the general Formula I are salts formed with nontoxic organic acids, e.g. maleic, fumaric, benzoic, ascorbic, oxalic, tartaric, lactic and malic acid, or inorganic acids, e.g. hydrochloric, hydrobromic, sulfuric, phosphoric and nitric acid or they may be salts of inorganic bases such as alkali metal salts, e.g. sodium, potassium, or lithium salts, alkaline earth metal salts, e.g. calcium or magnesium salts, or ammonium salts or salts of organic bases.

In Formula I, A is preferably a bond or C₁-C₃ alkylene.

B is preferably -CH(NR'R'')-COOH wherein R' and R'' are hydrogen or a group of Formula II wherein R₂, R₃ and R₄ are hydrogen or lower alkyl, or R₄ and R₂ are connected in order to form a C₁-C₃ alkylene group. Most preferably, B is -CH( NH₂)-COOH or a group of Formula II wherein each of R_{2,} R₃ and R₄ are hydrogen.

Preferably, E is O, COO, -O-(CH₂)ₙ-COO (n = 1, 2 or 3) or tetrazolyl and D is oxygen.

Particularly suitable R₁ groups are thienyl, substituted thienyl, oxazolyl, isoxazolyl, thiazolyl, isothiazolyl, pyrazolyl, imidazolyl, oxadiazolyl, substituted oxadiazolyl, thiadiazolyl, tetrazolyl, triazolyl, pyridyl, phenyl, biphenyl and naphthyl. Preferred groups are 2-thienyl, 3-thienyl, phenyl, 2-pyridyl and 4-pyridyl, and 2-thienyl and phenyl substituted with halogen or methyl.

In a preferred embodiment of the invention A is a bond or C₁-C₃ alkylene, B is -CH(NH₂)-COOH or a group of Formula II wherein each of R₃, R₄ and R₂ are hydrogen, E and D are both oxygen, and R₁ is 2-pyridyl, 4-pyridyl, thienyl, phenyl, substituted thienyl or substituted phenyl.

According to the invention the novel compounds of formula I, are prepared by a method comprising:
a) in order to obtain a compound of Formula I wherein B is a-CH(NR'R'')-COOH group wherein R' and R'' are as previously defined, deprotection of a compound of the general Formula III 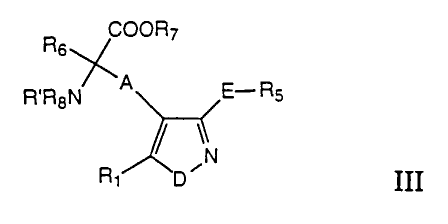 wherein R₁, R', A, D and E are as previously defined, R ₅, R₇ and R₈ are protection groups, and R₆ is hydrogen or a protection group;
b) in order to obtain a compound of Formula I wherein B is a -CH(NR'R'')-COOH group wherein R' and R'' are both hydrogen, deprotection of a compound of the general Formula IV 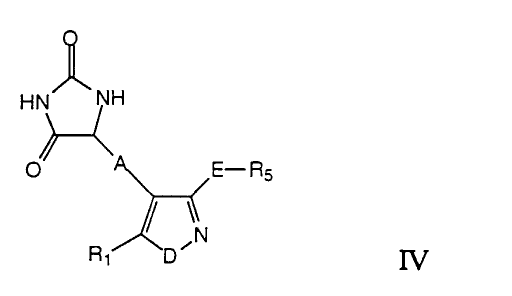 wherein R₁, R₅, A, D and E are as previously defined;
c) in order to obtain a compound of Formula I wherein B is a group of Formula II, addition-elimination reaction of a compound of the general Formula V with a compound of the general Formula VI: 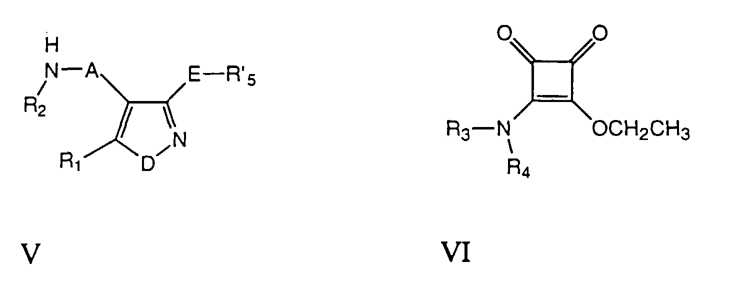 in which formulas R₁ - R₄, A, D and E are previously defined and R' ₅ is hydrogen or a protecting group;
d) In order to obtain a compound of Formula I, wherein B is a group of Formula II wherein R⁴ and R² are linked to form a C₁₋₃ alkylene, C₂-C₃ alkenylene or C₂-C₃ alkynylene group optionally mono- or di-substituted with hydroxy or methyl, reacting a compound of Formula VII 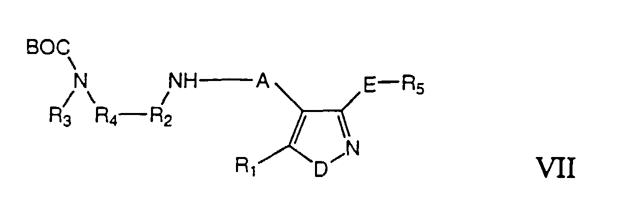 wherein R₁, R₃, A, D and E are as previously defined; R₄ and R₂ are linked to form a group as defined above and BOC is t-butoxycarbonyl, with 3,4-diethoxy-3-cyclobuten-1 ,2-dion and subsequent ringclosure and deprotection;
e) in order to obtain a compound of Formula I wherein B is a group of Formula II and one or more of R₂ - R₄ are different from hydrogen, alkylation of a compound of the general Formula VIII 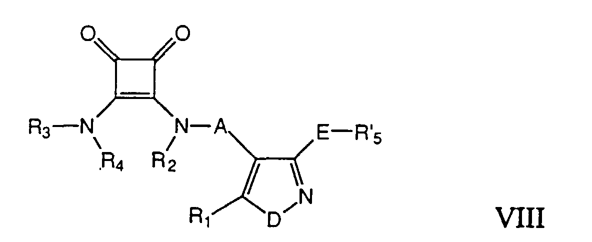 wherein R₁, R₂, R₃, R₄, A, D and E are as previously defined, at least one of R₂ - R₄ however being hydrogen and R'₅ is hydrogen or a protection group.

In the method of the invention preferred protection groups are as follows:
R₅ and R'₅: lower alkyl, benzyl or a benzenesulfonyl group; R₆: lower alkoxycarbonyl, R₇: lower alkyl and R₈: lower alkylcarbonyl.

The one step deprotection according to method a) is carried out by treatment of the compound of Formula III with a suitable aqueous acid, conveniently in an aqueous solution of 48 % HBr, a saturated solution of HBr i acetic acid or a 2-12 N aqueous solution of HCl. The deprotection may also be carried out in successive steps by using aqueous acids and aqueous bases, conveniently successively in an aqueous acid such as 1-12 N HCl, an aqueous base such as 1-8 N NaOH and an aqueous acid such as 1-12 N HCl, or successively in an aqueous base such as 1-8 N NaOH and an aqueous acid such as 1-12 N HCl. When R₅ is benzyl the deprotection of the E-group is alternatively accomplished by catalytic hydrogenation, conveniently by using palladium as a catalyst, either before or after the deprotection of the α-amino acid.
The starting materials of general Formula III are conveniently obtained as described by Christensen, I.T. et al, *Drug Design and Delivery* **1989**, *5*, 57-71 and Christensen, S.B. *et al, Acta. Chem.Scand.* **1978**, *B 32*, 27-30. Starting materials of Formula III wherein E is COO, (CH₂)ₙ-COO, O-(CH₂)ₙ-COO, S-(CH₂)ₙ-COO wherein n is 1-6, a 5-tetrazolyl-C₁₋₆ alkyl group or a 3-hydroxyisoxazolyl-C₁₋₆ alkyl group are conveniently obtained as described in Krogsgaard-Larsen, P., et al., *J. Med. Chem.* **1991**, *34*, 123-130, Madsen, U., *Bio. Med. Chem. Lett.* **1993**, *8*, 1649-1654 and Madsen, U. and Wong, E. *J Med. Chem.* **1992**, *35*, 107-111.

Starting materials of Formula III wherein R₁ is a heteroaryl group, which is unstable in acidic environment, are conveniently prepared from (3-alkoxy-4-methylisoxazol-5-yl)carboxylic acid by formation of the heteroaryl in the 5-position, bromination of the 4-methylisoxazole group and subsequently alkylation with an amino acid precursor *e.g.* diethyl acetamidomalonate. The alkyl protected 3-hydroxyisoxazole group may or may not be reprotected with a suitable protection group like *e.g.* the benzenesulfonyl group before, during or after the synthesis of the heteroaryl in the 5-position of the isoxazole. (3-Alkoxy-4-methylisoxazol-5-yl)carboxylic acid is conveniently obtained from 3-alkoxy-4,5-dimethylisoxazole prepared as described by Hansen, J. J., *J. Chem. Soc., Perkin Trans. 1*, **1980**, 1826-33, by bromination and subsequently oxidation of the 5-methylisoxazole group to the corresponding 5-isoxazolcarboxylic acid compound.
In other cases starting materials of Formula III wherein R₁ is *e.g.* a pyridyl group are conviently obtained by a modification of the method described by K. Tomita, *Ann. Sankyo Res. Lab.*, **1973**, *25*, 3-5. Protection of the 3-hydroxy group in the 3-hydroxy-5-arylisoxazole obtained followed by introduction of a hydoxymethyl group in the 4-position of the isoxazole ring leads to an intermediate which is readily converted to the starting material of Formula III.

In b) the one step deprotection is carried out by treatment of a compound of Formula IV with a suitable aqueous acid or aqueous base, conveniently in 2-8 N aqueous hydrochloric acid. The deprotection may also be performed in successive steps by using aqueous acids and aqueous bases as mentioned above for method a). The hydantoin ring may also be cleaved by the use of a aqueous solution of Ba(OH)₂, aqueous 10-70 % sulphuric acid or by the use of enzymes such as hydantoinases. The cleavage of the hydantoin ring may be carried out either before or after the deprotection of the E-group.
The hydantoin rings in the compounds of the general Formula IV are conveniently formed according to the methods described by Ware, E.,*Chem.Rev.* **1950**, *46*, 403-470. The cleavage of the hydantoin ring is conveniently performed in analogy with the methods described by Curry, K. *et al J.Med.Chem.* **1988**, *31*, 864-867, Farrington, G.K. *et a!*, *J.Med.Chem.* **1987**, *30*, 2062-2067, Grunewald, G.L. *et al*, *J.Med.Chem.* **1980**, *23*, 754-758, Hiroi, K. *et al, Chem.Pharm.Bull.* **1968**, *1 6*, 444-447 or Stark, G.R. *et al, J.Biol.Chem.* **1963**, *238*, 214-226.
The starting material for preparation of compounds of Formula IV may be obtained in analogy with the method described by Madsen, U., *Eur. J Med. Chem.* **1993**, *28*, 791-800.
When R₅ is benzyl the deprotection of the E-group is conveniently performed by hydrogenation using palladium as a catalyst.

The addition-elimination reaction according to method c) is conveniently performed in a protic organic solvent such as an alcohol, preferably in the presence of a suitable inorganic base such as aqueous NaOH at room temperature. The intermediates of Formula VI may be prepared by the methods described by Cohen, S. *et al, J.Amer.Chem.Soc.* **1966**, *88*, 1533-1536, EP-A2-0496561 or Kinney, W.A. *et al, J.Med.Chem.* **1992**, *35*, 4720-4726.

The intermediate of the general formula V is readily obtained by a Gabriel synthesis of primary amines as described by Sheehan, J. C. *et a!., J. Am. Chem. Soc.,* **1950**, *72*, 2786-88. The alkyl halogenide starting materials for this synthesis is conviently obtained as described with respect to starting materials used in method a), cf. above.

The deprotection is conveniently preformed by the use of an aqueous acid or an aqueous base, preferably 0.5-8 N HCl or aqueous 0.5-8 N NaOH, either at room temperature or at elevated temperatures. When R' ₅ is benzyl the deprotection is alternatively performed by hydrogenation using palladium as a catalyst.

In method d) the reaction and the subsequent ringclosure and deprotection are performed as described by Kinney et al., EP-A2-0496561.

The starting materials of formula VII may be obtained by reacting e.g. 4-bromomethyl isoxazole obtained as described with respect to the starting materials in method a) with a mono-BOC-proteted alkylene diamine cf. EP-A2-0496561. The alkylation of compounds of the general Formula VIII according to method e) is conveniently performed in an inert organic solvent such as a suitable alcohol, ketone or dimethylformamide preferably in the presence of a suitable base such as sodium hydride, potassium carbonate or triethylamine, as described by Kinney, W.A., EP-A2-0496561. The starting materials of formula VIII are obtained by method c).

When β-keto esters are used as starting materials for the formation of 3-hydroxy-isoxazoles, which again are used as starting material for obtaining compound III-V and VII-VIII, are either commercially avaible or they may conveniently be prepared according to the methods described by Cason, J. *et al*, *J.Org.Chem.* **1953**, *18*,1594-1600 and Hannick, S.M. *et al*, *J.Org.Chem.* **1983**, *48*, 3833-3835. Corresponding isothiazole starting materials may be prepared according to the methods described in EP-A1-0 336 555.

Resolution of the compounds of general formula I is conviently performed by diastereomeric saltformation using optical active acids or bases, e.g. 1-phenylethylamine. In some cases the resolution is conviently performed by formation of diastereomeric compounds and subsequently separation of the diastereomers by flash chromatography or crystallisation.

Salts of the compounds of the invention are easily prepared by methods well known in the art, i.e. by reacting the compound with either the equivalent amount of acid or base in an aqueous miscible solvent, such as acetone or ethanol, with isolation of the salt by concentration and cooling, or reacted with an excess of the acid or base in an aqueous immiscible solvent such as ethyl ether or chloroform, with the desired salt separating directly. These salts may also be prepared by the classical method of double decomposition of appropriate salts.

The compounds of general Formula I and the pharmaceutically acceptable acid addition salts thereof may be administered in any suitable way, e.g. orally or parenterally, and the compounds may be presented in any suitable form for such administration, e.g. in the form of tablets, capsules, powders, syrups or solutions or dispersions for injection.

An effective daily dose of a compound of general Formula I or a pharmaceutically acceptable salt thereof is from 10 µg/kg to 50 mg/kg body weight.

### Examples

In the following the invention is further illustrated by way of examples which may in no way be construed as limiting for the invention.

All melting points were determined on a Büchi SMP-20 apparatus and are uncorrected. ¹H NMR and ¹³C NMR spectra were recorded on a Brucker 250 MHz spectrometer (250.13 MHz for ¹H NMR and 62.90 MHz for ¹³C NMR) using TMS as an internal standard if not otherwise stated. In case of the title compounds **13a**, **13b**, **14c** and **14d**, the ¹H NMR and ¹³C NMR spectra were recorded on a Brucker 200 MHz spectrometer (200.0 MHz for ¹H NMR and 50.3 MHz for ¹³C NMR) using TMS as an internal standard if not otherwise stated.

Mass spectra were obtained on a Quattro MS-MS system from VG Biotech, Fisons Instruments, Manchester, GB. The MS-MS system was connected to an HP 1050 modular HPLC system. A volume of 20 - 50 µl of the sample (0.1 - 0.05 mg/ml) dissolved in a mixture of acetonitrile/water/conc. aqueous ammonia (25 %) = 25:25:1 (v/v/v) was introduced via the autosampler at a flow of 30 µl/min into the Electrospray Source. Spectra were obtained at standard operating conditions to obtain molecular weight information (MH+). The background was subtracted. The enantiomeric excess (ee) of the enantiomeric compounds were determined by chiral HPLC using a chiral crown ether column. The chiral HPLC was performed on a 150- x 4-mm Crownpak CR(-) or Crownpak CR(+) column (Daicel) eluted at 15-40 °C with 0.4-1.0 mL/min. of aqueous perchloric acid/methanol (100-85 %/0-15 %). One of the following instrumentations were used:
1) Jasco 880-PU pump, a Rheodyne 7125 injector, and a Waters 480 UV detector, set at 210 nm, connected to a Merck-Hitachi D-2000 Chromato-Integrator.
2) Hitachi-Merch L-6200 pump, a Hitachi-Merch 655A-40 autosampler and a Hitachi-Merch L-4000 UV detektor, set at 205 nm, connected to a Hitachi-Merch D-2500 Integrator.
The enantiomeric purities were calculated from peak areas.

### EXAMPLE 1

### (RS)-2-Amino-3-[3-hydroxy-5-(2-thienyl)isoxazol-4-yl]propionic Acid, 1a.

A mixture of 2-bromothiophene (250.0 g, 1.53 mol) and CuCN (157.5 g, 1.76 mol) was boiled under reflux in NMP (625 mL) for 90 mm. The mixture was cooled to 100 °C and poured onto a hot solution of NaCN in water (150 g of NaCN in 2.5 L of water). The mixture was stirred vigorously at 80 ° C for 30 min, and filtered hot. The cooled mixture was extracted with diethyl ether (3 x 2 L) and reduced *in vacuo* to 1.5 L. The organic phase was washed with water (750 mL) and an aqueous saturated solution of NaCl (750 mL). The organic phase was dried (MgSO₄) and concentrated i*n vacuo* to give a red/violet oil, which was distilled under reduced pressure (1 mmHg) to give 2-thiophenecarbonitrile (105.3 g, 63%).

A mixture of 2-thiophenecarbonitrile (25.0 g), activated zinc (22.5 g) and CuBr₂ (0,2 g; 0.9 mmol) in benzene (350 mL) was heated to reflux temperature (83 °C). Ethyl 2-bromopropionate (62.2 g) in benzene (150 mL) was added over 60 min. at 83 °C. The resulting mixture was refluxed for 2 h and cooled to 0 °C. 15 % Aqueous H₂SO₄ (400 mL) was added over 60 mm. (temp. < 10 °C) and the mixture was stirred for 20 h at 20 °C. The mixture was filtered and the phases were separated. The aqueous phase was extracted with diethyl ether (2 x 500 mL) and the combined organic phases were dried (MgSO₄) and evaporated *in vacuo.* Column chromatography (silica gel, eluent: ethyl acetate/*n*-heptane/methanol = 4:4:1) gave ethyl 2-methyl-3-(2-thienyl)-3-oxopropionate as an oil (35.0 g; 72 %).

A mixture of ethyl 2-methyl-3-(2-thienyl)-3-oxopropionate (25.3 g) and NaOH (5.0 g; 0.12 mol) in methanol/water (10:1, 200 mL) was cooled to -30°C. An ice-cooled (0 °C) filtered solution of NH ₂OH, HCl (16.6 g) and NaOH (10.0 g) in methanol/water (10:1, 200 mL) was added and the resulting solution was stirred for 3 h at -30 °C. The solution was allowed to reach 5 °C and then added to conc. HCl (280 mL) at 80 °C over 45 min. The resulting mixture was refluxed for 1 h at 80 °C. The methanol was evaporated and water was added (250 mL). The solution was cooled to 5 °C and the resulting crystals were collected by filtration. The crystals were dissolved in CH₂Cl₂ (500 mL). The organic phase was dried (MgSO₄) and evaporated *in vacuo* to give 4-methyl-5-(2-thienyl)isoxazol-3-ol (12.0 g; 55 %).

A suspension of 4-methyl-5-(2-thienyl)isoxazol-3-ol (12.5 g) and K₂CO₃ (14.4 g) in acetone (250 mL) was heated to reflux temperature. Ethylbromide (8.0 g) was added over 25 min. at reflux temperature and the resulting mixture was refluxed for 3 h. Additional ethylbromide (8.0 g) was added in one portion and the resulting mixture was refluxed for further 3 h. After filtration and removal of the solvent the residue was subjected to column chromatography (silica gel, eluent: ethyl acetate/ *n*-heptane = 1:1) and 3-ethoxy-4-methyl-5-(2-thienyl)isoxazole was isolated as an oil (7.3 g; 51 %).

A mixture of 3-ethoxy-4-methyl-5-(2-thienyl)isoxazole (5.1 g) and *N*-bromosuccinimide (NBS) (5.1 g) in CCl₄ (400 mL) was refluxed for 18 hours. Filtration and removal of the solvent gave 4-bromomethyl-3-ethoxy-5-(2-thienyl)isoxazole (7.8 g; 100 %).

To a mixture of diethyl acetamidomalonate (4.6 g) and potassium *tert*-butoxide (2,4 g) in *N*-methyl-2-pyrrolidone (NMP) (100 mL) was added a solution of 4-bromomethyl-3-ethoxy-5-(2-thienyl)isoxazole (3.0 g) in NMP (25 mL) at 22 °C. The resulting mixture was stirred for 1 h at 22 °C and poured onto a water/ice mixture. The aqueous phase was extracted with diethyl ether and the combined organic phases were washed with an aqueous saturated solution of NaCl. The organic phases were dried (MgSO₄) and evaporated *in vacuo*. The residue was subjected to column chromatography (silica gel, eluent: ethyl acetate/*n*-heptane/methanol = 5:5:1) which gave ethyl 2-acetylamino-2-ethoxycarbonyl-3-[3-ethoxy-5-(2-thienyl)isoxazol-4-yl]propionate (3.0 g; 68 %).

A mixture of 2-acetylamino-2-ethoxycarbonyl-3-[3-ethoxy-5-(2-thienyl)isoxazol-4-yl]propionate (2.7 g) and HBr 48 % (20 ml) was refluxed for 1 h. The mixture was evaporated *in vacuo* and the residue was dissolved in water (50 mL) and treated with charcoal. After filtration the pH was adjusted to 3 with 4 N aqueous NaOH. The resulting crystals were collected by filtration and dried *in vacuo* to give the title compound, **1a**, (0.5 g; 30 %). Mp. 237-39 °C (dec.), CHN; Calcd: 47.23; 3.97; 11.02, Found: 47.21; 4.02; 10.92.
¹H NMR (DMSO-*d*₆): δ 9.90 (b, 1H), 7.82 (dd, 1H), 7.55 (dd, 1H), 7.24 (dd, 1H), 3.70 (dd, 1H), 2.95-2.88 (m, 2H).
¹³C NMR (DMSO-*d*₆): δ 171.42, 171.23, 159.62, 129.27, 128.65, 128.22, 127.15, 101.89, 52.59, 24.99.

The following compound was prepared in a similar manner:

### (RS)-2-Amino-3-[3-hydroxy-5-(3-thienyl)isoxazol-4-yl]propionic Acid, 1b, Mp: 239-40 °C (dec.).

¹H NMR (DMSO-*d*₆): δ 8.03-7.96 (m, 1H), 7.76-7.70 (m, 1H), 7.48-7.43 (m, 1H), 3.70-3.63 (m, 1H), 3.00-2.76 (m, 2H).

The following compounds was prepared in a similar manner using the benzenesulfonyl group as a protection group for the 3-hydroxyisoxazole group:

### (RS)-2-Amino-3-[3-hydroxy-5-(2-naphthyl)isoxazol-4-yl]propionic Acid, Hydrate, 1c. Mp: 235-37 °C (dec.)

¹H NMR (DMSO-*d*₆): δ 2.89-3.16 (m, 2H); 3.70-3.77 (m, 1H); 7.56-7.63 (m, 2H); 7.74 (dd, 1H); 7.92-8.09 (m, 3H); 8.22 (s, 1H).
¹³C NMR (DMSO-*d*₆): δ 25.10; 53.03; 103.03; 124.30; 125.90; 126.89; 126,96; 127.45; 127.79; 128.70 (2C); 132.74; 133.18; 164.67; 171.41; 171.54
**MS** (MH+) *m/z*: 299.

### (RS)-2-Amino-3-[3-hydroxy-5-(4-trifluoromethylphenyl)isoxazol-4-yl]propionic Acid, 1d. Mp: 237-39 °C (dec.)

¹H NMR (DMSO-*d*₆): δ 2.80 (dd, 1H); 2.97 (dd, 1H); 3.71 (dd, 1H); 7.87 (S, 4H)
¹³C NMR (DMSO-*d*₆, 5 % CF₃COOH) δ 23.35; 50.94; 101.72; 121.94; 126.11; 126.16; 127.77 (2C); 130.34 (q, CF₃); 131.74; 164.13; 170.29 (2C).
**MS** (MH+) *m/z*: 317.

### (RS)-2-Amino-3-[3-hydroxy-5-(3-benzo[b]thienyl)isoxazol-4-yl]propionic Acid, Hemihydrate, 1e. Mp: 222-24 °C (dec.)

¹H NMR (DMSO-*d*₆): δ 2.75 (dd, 1H); 2.96 (dd, 1H); 3.70 (dd, 1H); 7.43-7.55 (m, 2H);7.96-8.03 (m, 1H); 8.05-8.14 (m, 1H); 8.21 (s, 1H).
¹³C NMR (DMSO-*d*₆): δ 25.02; 52.95; 104.05; 123.17; 123.34; 123.67; 125.27(2C); 129.49; 136.80; 139.35; 161.49; 171.11; 171.31.
**MS** (MH+) *m/z*: 305.

The following compound was prepared in a similar manner from 2-acetylamino-2-ethoxycarbonyl-3-[3-ethoxy-5-(2-thienyl)isoxazol-4-yl]propionate by lithiation and methylation of the 5-position of the thienyl group and subsequently deprotection by boiling HBr 47 % (aq).

### (RS)-2-Amino-3-[3-hydroxy-5-(5-methyl-2-thienyl)isoxazol-4-yl]propionic Acid, Hydrate, 1f. Mp: 242-44 °C.

¹H NMR (DMSO-*d*₆): δ 2.51 (s, 3H); 2.84-2.93 (m, 2H); 3.63-3.72 (m, 1H); 6.94 (dd, 1H);7.33 (d, 1H).
¹³C NMR (DMSO-*d*₆): δ 14.91; 24.95; 52.62; 101.24; 126.63; 126.88; 127.23; 142.33; 159.71; 171.20; 171.31.
**MS** (MH+) *m/z*: 269.

### EXAMPLE 2

### (RS)-2-Amino-2-(3-hydroxy-5-phenylisoxazol-4-yl)acetic Acid, 2a.

A mixture of 3-ethoxy-4-methyl-5-phenylisoxazole (3.0 g; 15 mmol) prepared as described by Christensen, I.T. **1989**, *supra*, NBS (5.5 g; 31 mmol) and dibenzoylperoxide (0.2 g; 0.8 mmol) in CCl₄ (100 mL) was refluxed for 20 h. The mixture was cooled to ambient temperature, filtered and evaporated *in vacuo*. The residue was dissolved in water (95 mL) and refluxed for 20 h. After cooling the water phase was extracted with diethyl ether (3 x 100 mL). The combined organic phases were dried (Na₂SO₄) and evaporated *in vacuo* to give 4-(3-ethoxy-5-phenylisoxazol)carbaldehyde (2.4 g; 75 %).

A mixture of 4-(3-ethoxy-5-phenylisoxazol)carbaldehyde (1.9 g; 8.7 mmol), KCN (2.7 g; 40.5 mmol) og (NH₄)₂CO₃ (7.8 g; 81.1 mmol) in 50 % aqueous methanol (250 mL) was refluxed for 6 h. The methanol was evaporated *in vacuo* and the aqueous phase was extracted with ethyl acetate (3 x 150 mL). The combined organic phases were dried (Na₂SO₄) and the solvent removed by evaporation *in vacuo*. The residue was subjected to column chromatography (silica gel, eluent ethyl acetate/*n*-heptane/methanol = 5:5:1) to give 3-ethoxy-4-[5-(imidazolidin-2,4-dione)]-5-phenylisoxazole (1.0 g; 40 %).

A suspension of 3-ethoxy-4-[5-(imidazolidin-2,4-dione)]-5-phenylisoxazole (800 mg; 2.8 mmol) in 6 N aqueous HCl (20 mL; 120 mmol) was refluxed for 48 h. The mixture was evaporated *in vacuo* and the residue was dissolved in water (200 mL) and triethylamine (TEA) (870 mg; 8.5 mmol). Di-*tert*-butyl dicarbonate (930 mg; 4.3 mmol) in tetrahydofuran (THF) (50 ml) was added and the resulting solution was stirred for 20 h at 20 °C. The THF was evaporated *in vacuo* and pH was adjusted to 6.5 with 0.1 N aqueous HCl. The aqueous phase was extracted with diethyl ether which was discharged. pH in the aqueous phase was adjusted to 2 with 0.1 N aqueous HCl and the aqueous phase was extracted with diethyl ether (3 x 100 mL). The combined organic phases were dried (Na₂SO₄) and evaporated *in vacuo*. The residue was dissolved in diethyl ether (40 mL) and a saturated solution of HCl in diethyl ether was added. The resulting mixture was stirred for 20 h at 20 °C. The precipitate was collected by filtration and dried. The crystals were dissolved in water (5 mL) and pH was adjusted to 3. The precipitate was collected by filtration and dried to give the title compound, **2a**, (100 mg; 15 %). Mp. 228-230 °C (dec.). CHN; Calc: 56.40; 4.31; 11.96, Found: 56,20; 4.37; 11.74.
¹H NMR and ¹³C NMR data for **2a**, HCl:
¹H NMR (DMSO-*d*₆): δ 7.83-7.68 (m, 2H), 7.64-7.40 (m, 3H), 5.12-4.95 (m, 1H), 3.90 (broad).
¹³C NMR (DMSO-*d*₆): δ 169.10, 168.38, 168.31, 131.39, 129.60(2C), 127.73(2C), 126.95, 99.69, 46.06.

The following compounds were prepared in a similar manner with preparation of the 4-isoxazolecarbaldehydes from the corresponding 4-isoxazolebromomethyl compounds using hot (115 °C) dimethylsulfoxide and sodiuinhydrogencarbonate:

### (RS)-2-Amino-2-[3-hydroxy-5-(2-thienyl)isoxazol-4-yl]acetic Acid, 2b. Mp: 191-193 °C (dec.).

¹H NMR (DMSO-*d*₆) δ 4.50 (s, 1 H), 7.26 (dd, 1 H), 7.77 (d, 1 H), 7.84 (d, 1 H).
¹³C NMR (HCl salt) (DMSO-*d*₆) δ 46.16, 98.87, 127.62, 129.15, 129.83, 131.20, 163.56, 168.45, 169.39.
**MS** (MH+) *m/z*: 241.

### (RS)-2-Amino-2-[3-hydroxy-5-(4-trifluormethylphenyl)isoxazol-4-yl]acetic Acid, Hydrate, 2c. Mp: 200-201 °C.

¹H NMR (DMSO-*d*₆): 4.46 (s, 1H); 7.92 (d, 2H); 8.11 (d, 2H).
¹³C NMR (HCl salt) (DMSO-*d*₆): 45.84; 101.16; 121.98; 126.43; 126.50; 128.76 (2C); 130.75; 131.00 (q, CF₃); 166.75; 168.20; 169.21.
**MS** (MH+) *m/z*: 303.

### EXAMPLE 3

### (RS)-2-Amino-5-(3-hydroxy-5-phenylisoxazol-4-yl)pentanoic Acid, hydrate, 3a.

The title compound was prepared in analogy with the method described by Christensen, I.T. *et al*, *Drug Design and Delivery* **1989**, *5*, 57-71, with the following modifications 1) Ethyl benzoylacetate was used as starting material. 2) The isoxazole ring in the intermediate 5-phenyl-4-(2-propenyl)-3-isoxazolol was prepared according to the method of Sato, K. *et al*, *Agric. Biol. Chem.* **1986**, *50*, 1831-1837. 3) The zwitterion of the title compound **3a**, was obtained by adjusting pH in the aqueous phase to 3.5 with 0.1 N aqueous NaOH. Mp. 210-212 °C (dec.), CHN; Calcd: 54.62; 6.40; 9.10, Found: 54.67; 6.36; 9.13.
¹H NMR (DMSO-*d*₆): δ 7.72-7.61 (m, 2H), 7.59-7.43 (m, 3H), 3.64-3.40 (m, 2H), 3.50 (broad), 3.31-3.19 (m, 1H), 1.82-1.53 (m, 4H).
¹³C NMR (DMSO-*d*₆): δ 170.75, 163.44, 161.65, 129.72, 129.23(2C), 128.58, 126.19(2C), 105.68, 53.93, 30.57, 25.13, 20.88.

### EXAMPLE 4

### (RS)-2-Amino-4-[5-(4-fluorophenyl)-3-hydroxyisoxazol-4-yl]butanoic Acid, 4a.

The β-keto ester, ethyl 3-oxo-3-(4-fluorophenyl)propionate, was prepared in analogy with the method described in Example 1 using 4-fluorobenzonitrile (25.0 g; 0.21 mol), ethyl 2-bromacetate (51.7 g; 0.31 mol), activated zinc (20.3 g; 0.31 mol), CuBr₂ (0,2 g; 0.9 mmol) and benzene (500 ml). Column chromatography (silica gel, eluent: ethylacetate/*n*-heptane/methanol = 5:5:1) of the residue gave ethyl 3-oxo-3-(4-fluorophenyl)propionate as an oil (28.0 g; 65 %).

To a solution of NaH (4.4 g; 0.15 mol, 80 % in mineral oil) in ethanol (500 mL) was added ethyl 3-oxo-3-(4-fluorophenyl)propionate (28.0 g; 0.13 mol). The resulting solution was stirred for 1.5 h at 25 °C. Ethyl 3-chloropropionate (20.0 g; 0.15 mol) was added over 30 min. at 25 °C and the mixture was refluxed for 20 h. The solvent was removed by evaporation and the residue was dissolved in water (400 mL) and extracted with ethyl acetate. The combined organic phases were dried (MgSO₄) and evaporated *in vacuo*. Column chromatography (silica gel, eluent: ethylacetate/*n*-heptane/methanol = 4:4:1) of the residue gave ethyl 4-ethoxycarbonyl-5-(4-fluorophenyl)-5-oxopentanoate (27.5 g: 67 %).

Ethyl 3-[5-(4-fluorophenyl)-3-hydroxyisoxazol-4-yl]propionate was prepared in analogy with the the method described in Example 1 with the following modifications: 1) Ethyl 4-ethoxycarbonyl-5-(4-fluorophenyl)-5-oxopentanoate (27.5 g; 89 mmol) was used as starting material. 2) After evaporation of the methanol from the methanol/water solution, the aqeous phase was extracted with diethyl ether. The combined organic phases were dried (MgSO₄) and evaporated *in vacuo*. The residue was dissolved in ethanol (400 mL) and acetyl chloride (40 mL) was added. The resulting mixture was refluxed for 20 h, cooled and the solvent was removed by evaporation *in vacuo*. Column chromatography (silica gel, eluent: ethyl acetate/-*n*-heptane/methanol = 2:2:1) of the residue gave ethyl 3-[5-(4-fluorophenyl)-3-hydroxyisoxazol-4-yl]propionate (9.3 g; 31 %).

Ethyl 3-[3-ethoxy-5-(4-fluorophenyl)isoxazol-4-yl]propionate was prepared as described in Example 1 with the following modifications: 1) Ethyl 3-[5-(4-fluorophenyl)-3-hydroxyisoxazol-4-yl]propionate (5.5 g; 20 mmol) was used as starting material. 2) Instead of two equivalents, only one equivalent of ethylbromide was added. Column chromatography (silica gel, eluent: ethyl acetate/*n*-heptane/ methanol = 10:10:1) of the residue gave ethyl 3-(3-ethoxy-5-(4-fluorophenyl)isoxazol-4-yl)propionate (4.0 g; 65 %).

3-[3-Ethoxy-5-(4-fluorophenyl)isoxazol-4-yl]propanal was prepared from ethyl 3[3-ethoxy-5-(4-fluorophenyl)isoxazol-4-yl]propionate according to the method described by Rich, D.H. *et al, J.Org.Chem*. **1978**, *43*, 3624-3626.

3-Ethoxy-5-(4-fluorophenyl)-4-[2-[5-(imidazolidin-2,4-dione)]ethyl]isoxazole was prepared as described in Example 2 using the corresponding aldehyde as starting material. The crude product obtained was recrystallized twice from ethanol to give the pure substance.

The title compound, **4a**, was prepared from the hydantoin in analogy with the method described in Example 2 in a yield of 19 %. Mp. 235-237 °C (dec.). CHN; Calcd: 55.71; 4.68; 10.00, Found: 54.59; 4.61; 9.97.
¹H NMR (DMSO-*d*₆, 340 °K): δ 7.78-7.54 (m, 2H), 7.39-7.19 (m, 2H), 5.92 (b), 3.35-3.18 (m, 1H), 2.81-2.53 (m, 2H), 2.04-1.70 (m, 2H).
¹³C NMR (DMSO-*d*₆): δ 172.19, 170.79, 164.70 and 160.76 (C-F), 162.96, 128.64, 128.50, 125.13, 116.55, 116.21, 104.52, 52.35, 31.44, 17.81.

The following compounds were prepared in a similar manner:

### (RS)-2-Amino-4-(3-hydroxy-5-phenylisoxazol-4-yl)butanoic Acid, hydrochloride, 4b. Mp. 230-233 °C (dec.).

¹H NMR (D₂O): δ 7.59-7.48 (m, 2H), 7.47-7.32 (m, 3H), 3.61-3.50 (m, 1H), 2.57-2.41 (m, 2H), 2.08-1.90 (m, 2H).
¹³C NMR (DMSO-*d*₆): δ 179.12, 176.64, 161.12, 130.41, 128.79(2C), 128.13, 125.72(2C), 107.13, 53.31, 32.97, 18.62.

### (RS)-2-Amino-4-[3-hydroxy-5-(2-thienyl)isoxazol-4-yl]butanoic Acid, hydrate, 4c. Mp. 214-216 °C (dec.).

¹H NMR (DMSO-*d*₆, 320 °K): δ 7.84-7.73 (m, 1H), 7.59-7.45 (m, 1H), 7.29-7.18 (m, 1H), 3.50 (very broad), 3.32-3.10 (m, 1H), 2.93-2.60 (m, 2H), 2.00-1.75 (m, 2H).
¹³C NMR (DMSO-*d*₆): δ 172.11, 170.64, 159.47, 129.41, 128.41(2C), 126.35, 103.73, 52.42, 30.76, 17.93.

### (RS)-2-Amino-4-[3-hydroxy-5-(3-thienyl)isoxazol-4-yl]butanoic Acid, 4d. Mp. 212-14 °C.

¹H NMR (DMSO-*d*₆): δ 8.02-7.95 (m, 1H), 7.78-7.72 (m, 1H),7.48-7.43 (m, 1H), 3.28-3,16 (m, 1H), 2.93-2.77 (m, 1H), 2.75-2.54 (m, 1H), 2.00-1.75 (m, 2H).

### (RS)-2-Amino-4-[3-hydroxy-5-(2-naphthyl)isoxazol-4-yl]butanoic Acid, Hydrate, 4e. Mp: 209-11 °C.

¹H NMR (DMSO-*d*₆): δ 1.87-2.05 (m, 2H); 2.60-2.85 (m, 2H); 2.90-3.06 (m, 1H); 7.56-7.66 (m, 2H); 7.80 (dd, 1H); 7.93-8.16 (m, 3H); 8.26 (s, 1H).
¹³C NMR (DMSO-*d*₆): δ 17.92; 31.50; 52.40; 105.02; 123.21; 125.66; 125.95; 127.04; 127.44; 127.76; 128.75; 128.91; 132.82; 133.09; 163.71; 170.70; 172.30.
**MS** (MH+) *m/z*: 313.

### EXAMPLE 5

### 4-[(2-Amino-3,4-dioxo-1 -cyclobuten-1 -yl)aminomethyl]-3-hydroxy-5-phenylisoxazole, hemihydrat, 5a.

To a mixture of potassium phthalimide (1.0 g; 5.4 mmol) in DMF (50 mL) was added 4-bromomethyl-3-ethoxy-5-phenylisoxazole (1.4 g; 5.0 mmol) in DMF (25 mL) at 90 °C 4-Bromomethyl-3-ethoxy-5-phenylisoxazole was prepared in analogy with the above mentioned method used for the preparation of 4-bromomethyl-3-ethoxy-5-(2-thienyl)isoxazol. The resulting mixture was stirred for 40 min. at 90 °C. CH₂Cl₂ (200 mL) was added and the mixture was poured onto water. The phases were separated and the water phase was extracted with diethyl ether. The combined organic phases were washed with 0.1 N aqueous NaOH and water, dried (Na₂SO₄) and evaporated *in vacuo* to give *N*-[(3-ethoxy-5-phenylisoxazol-4-yl)methyl]phthalimide (1.5 g; 88 %)

A suspension of *N*-[(3-ethoxy-5-phenylisoxazol-4-yl)methyl]phthalimide (1.3 g; 3.7 mmol) in aqueous 48 % HBr (20 mL) and acetic acid (20 mL) was stirred for 6 h at 110 °C. The mixture was evaporated *in vacuo* and the residue was dissolved in water. The aqueous phase was extracted with diethyl ether which was discharged. The water phase was evaporated *in vacua* and acetone (5 mL) was added to the residue. The precipitate was collected by filtration and dried to give 4-aminomethyl-5-phenyl-3-isoxazolol, hydrobromide (700 mg; 70 %).

To a solution of 3-amino-4-ethoxy-3-cyclobuten-1,2-dion (365 mg) prepared according to the method described by Cohen, S. *et al, J.Amer.Chem.Soc.* **1966**, *88*, 1533-1536 and 4-aminomethyl-5-phenyl-3-isoxazolol, hydrobromide (700 mg; 2.6 mmol) in ethanol (75 mL) was added a solution of NaOH (210 mg; 5.2 mmol) in water (5 mL) at 22 °C. The resulting mixture was stirred for 4 h at 22 °C. The solvent was evaporated and the residue was dissolved in water (200 mL). pH in the water phase was adjusted to 8.5. The aqueous phase was extracted with diethyl ether which was discharged. pH was adjusted to 3.75 and the precipitate was collected by filtration and dried to give the title compound **5a**, (675 mg; 92 %). Mp. 259-61 °C (dec.), CHN; Calcd: 57.13; 4.12; 14.28, Found: 57.70; 3.85; 14.29.
¹H NMR (DMSO-*d*₆): δ 7.81-7.24 (m, 5H), 4.78-4.56 (m, 2H), 3.5 (s, very broad).
¹³C NMR (DMSO-*d*₆): δ 183.34, 183.21, 170.14, 169.48, 168.29, 165.84, 130.58, 129.32(2c), 127.51, 126.72(2c), 102.52, 35.46.

The following compounds were prepared in a similar manner:

### 4-[(2-Amino-3,4-dioxo-1 -cyclobuten-1 -yl)aminomethyl]-3-hydroxy-5-(2-thienyl)isoxazole, 5b. Smp. 237-39 °C (dec.).

¹H NMR (DMSO-*d*₆): δ 7.88 (dd, 1H), 7.67 (dd, 1H), 7.26 (dd, 1H), 4.78-4.63 (m, 2H), 3.30 (s, very broad).
¹³C NMR (DMSO-*d*₆): δ 183.36, 183.18, 169.97, 169.51, 168.23, 161.29, 129.80, 128.59, 128.25, 127.82, 101.41, 35.20.

### 4-[(2-Amino-3,4-dioxo-1 -cyclobuten-1 -yl)aminomethyl]-3-hydroxy-5-(3-thienyl)isoxazole, 5c. Mp. 276-78 °C (dec.).

¹H NMR (DMSO-*d*₆): δ 8.16-8.13 (m, 1H), 7.82-7.77 (m, 1H), 7.56-7.50 (m, 1H), 4.76-4.66 (m, 2H).

### 4-[(2-Amino-3,4-dioxo-1 -cyclobuten-1 -yl)aminomethyl]-3-hydroxy-5-(4-trifluoromethylphenyl)isoxazole, 5d.

Mp: 269-72 °C
¹H NMR (DMSO-*d*₆): δ 4.72 (d, 2H); 7.93 (dd, 4H).
¹³C NMR (DMSO-*d*₆): δ 35.29; 104.19; 121.75; 126.12; 126.17; 127.60 (2C); 130.25 (q, CF₃); 131.11; 164.09; 168.21; 169.51; 170.11; 183.29 (2C).
**MS** (MH+) *m/z*: 354.

### EXAMPLE 6

### 4-[2-[(2-Amino-3,4-dioxo-1-cyclobutene-1-yl)amino]ethyl]-3-hydroxy-5-(2-thienyl)isoxazole, Hydrate, 6a.

A mixture of sodiumcyanide (4.0 g; 81 mmol) in DMSO (50 mL) was heated to 90 °C. 4-Bromomethyl-3-ethoxy-5-(2-thienyl)isoxazole (4.9 g; 16 mmol) dissolved in DMSO (50 mL) was added dropvise to the hot reaction mixture. The reaction mixture was stirred for additional 30 min. and was poured onto a water/ice mixture. The aqueous phase was extracted with CH₂Cl₂ and the combined organic phases were washed with an aqueous saturated solution of NaCl. The organic phases were dried (MgSO₄) and evaporated *in vacuo*. The residue was subjected to column chromatography (silica gel, eluent: ethyl acetate/*n*-heptane = 1:3) which gave (3-ethoxy-5-(2-thienyl)isoxazol)acetonitrile (2.2 g; 58 %).
A mixture of AlCl₃ (1.2 g; 9 mmol) in dry diethyl ether (50 mL) was added dropwise to a mixture of LiAlH₄ (0.34 g; 9 mmol) in dry diethyl ether (50 mL). The reaction mixture was stirred for additional 10 min. at room temperature. A mixture of [3-ethoxy-5-(2-thienyl)isoxazol]acetonitrile (2.0 g; 9 mmol) in dry diethyl ether (50 mL) was added to the stirred solution and the reaction mixture was stirred for additional 45 min. The reaction mixture was cooled to 5 °C and water (30 mL) and 6 M H₂SO₄ (5 mL) was added. pH in the acidic aqueous solution was raised to 11 using 6 M NaOH. The phases were separated and the aqueous phase was extracted with diethyl ether. The combined organic phases were dried (MgSO₄) and evaporated *in vacuo*. The residue was subjected to column chromatography (silica gel, eluent: ethyl acetate/*n*-heptane/methanol/triethylamine = 90:10:5:5) which gave 4-(2-amino-ethyl)-3-ethoxy-5-(2-thienyl)isoxazole (1.7 g; 76 %).
A mixture of 4-(2-aminoethyl)-3-ethoxy-5-(2-thienyl)isoxazole (1.7 g; 7 mmol) and HBr 47 % (aqueous) was refluxed for 1 h. The reaction mixture was evaporated *in vacuo* and acetone was added. The resulting crystals were collected by filtration and dried *in vacuo* to give 4-(2-aminoethyl)-3-hydroxy-5-(2-thienyl)isoxazole, hydrobromide (1.9 g; 94 %).
To a solution of 3-amino-4-ethoxy-3-cyclobuten-1,2-dion (480 mg: 3.4 mmol) prepared as previously described and 4-(2-aminoethyl)-3-hydroxy-5-(2-thienyl)isoxazole, hydrobromide (1.0 g; 3.4 mmol) in ethanol (30 mL) was added a solution of NaOH (270 mg; 6.8 mmol) in water (5 mL) at 22 °C. The resulting solution was stirred at room temperature for 18 h. The reaction mixture was evaporated *in vacuo* and water and diethyl ether was added. The phases were separated and the aqueous phase was extracted with diethyl ether which was discharged. The aqueous phase was acidified using 2 M HCl to pH 2.5 and was left at 5 °C for 18 h. The formed crystals were collected by filtration and washed with water, acetone and diethyl ether. The crystals were dried *in vacuo* to give the title compound, **6a**, (906 mg, 87 %). Mp. 248-50 °C (dec.).
CHN; Calcd: 50.39; 3.75; 13.57, Found: 50.66; 3.72; 13.55.
¹H NMR (DMSO-*d*₆): δ 2.77 (dd, 2H); 3.51-3.78 (m, 2H); 7.22 (dd, 1H); 7.57 (d, 1H); 7.81 (d, 1H).
¹³C NMR (DMSO-*d*₆): δ 24.12; 42.06; 102.06; 126.65; 127.94; 128.53; 129.00; 160.41; 168.65; 169.41; 170.05; 182.82; 183.00.
**MS** (MH+) *m/z*: 306.

### EXAMPLE 7

### (RS)-2-Amino-3-[3-(carboxymethoxy)-5-(2-thienyl)isoxazol-4-yl]propionic Acid, Monohydrate, 7a.

A suspension of 4-methyl-5-(2-thienyl)isoxazol-3-ol (10.0 g, 55 mmol) prepared as previously described and K₂CO₃ (19.1 g, 138 mmol) in acetone (350 mL) was stirred at room temperature for 30 min and then heated to reflux temperature. Ethyl chloroacetate (17.6 mL, 166 mmol) in acetone (125 mL) was added over 45 min and the resulting mixture was boiled under reflux for 210 min. The mixture was cooled to 5 °C, filtered and concentrated *in vacuo*. The residue was dissolved in CH₂Cl₂ (500 mL), washed with water (2 x 500 mL) and an aqueous saturated solution of NaCl (500 mL). The organic phase was dried (MgSO₄) and concentrated *in vacuo*. Flash chromatography (silica gel, eluent: *n*-heptane/ethyl acetate/methanol = 20:10:1) followed by concentration *in vacuo* gave ethyl [4-methyl-5-(2-thienyl)-3-isoxazolyloxy]acetate (8.7 g, 59%).
A mixture of ethyl [4-methyl-5-(2-thienyl)-3-isoxazolyloxy]acetate (2.5 g, 9.4 mmol) and NBS (2.0 g, 11.2 mmol) in CCl₄ (125 mL) was boiled under reflux for 8 h. The mixture was cooled, filtered and concentrated *in vacuo* to give ethyl [4-bromomethyl-5-(2-thienyl)-3-isoxazolyloxy]acetate (2.8 g, 88%).
A mixture of diethyl acetamidomalonate (3.5 g, 16.2 mmol) and potassium *tert*-butoxide (0.9 g, 17.0 mmol) in NMP (30 mL) was stirred at room temperature (25 °C) for 30 min. Ethyl [4-bromomethyl-5-(2-thienyl)-3-isoxazolyloxy]acetate (2.8 g, 8.1 mmol) in NMP (5 mL) was added (temp. 25-28 °C) and the resulting mixture was stirred at 28 ° C for 1 h and poured onto a water/ice mixture (250 mL). The aqueous phase was extracted with diethyl ether (3 x 200 mL) and the combined organic phases were washed with an aqueous saturated solution of NaCl (200 mL), dried (MgSO₄) and concentrated *in vacuo*. Flash chromatography (silica gel, eluent: CH₂Cl₂/ethyl acetate = 7:1) gave ethyl 2-acetylamino-2-ethoxycarbonyl-3-[3-[(ethoxycarbonyl)methoxy]-5-(2-thienyl)isoxazol-4-yl]propionate (2.3 g, 59%). A suspension of ethyl 2-acetylamino-2-ethoxycarbonyl-3-[3-[(ethoxycarbonyl)methoxy]-5-(2-thienyl)isoxazol-4-yl]propionate (2.0 g, 4.1 mmol) in 1 M HCl (130 mL) was boiled under reflux for 24 h.The reaction mixture was cooled, extracted with CH₂Cl₂ (2 x 150 mL) and treated with charcoal. Concentration *in vacuo* gave the title compound as the hydrochloric acid salt (1.2 g, 86%). The title compound, **7a** was obtained by addition of water (1.0 g, 71%).
Mp 228-230 °C (dec.).
CHN; Calcd: 43.64; 4.27; 8.48, Found: 43.07; 4.17; 8.43.
¹H NMR (DMSO-*d*₆) δ 2.86-3.28 (m, 2 H), 3.79 (dd, 1 H), 4.69 (s, 2 H), 7.26 (dd, 1 H), 7.69 (dd, 1 H), 7.86 (dd, 1 H).
¹³C NMR (DMSO-*d*₆) δ 23.50, 52.42, 67.39, 100.45, 127.91, 128.23, 128.48, 129.47, 161.44, 169.63, 170.14, 170.57.
**MS** (MH+) *m/z*: 313.

The following compound was prepared in a similar manner using ethyl 4-bromobutyrate as an alkylating agent instead of ethyl chloroacetate:

### (RS)-2-Amino-3-[3-(carboxypropoxy)-5-(2-thienyl)-isoxazol-4-yl]propionic Acid, 7b. Mp: 197-198 °C (dec.).

CHN; Calcd: 49.41; 4.74; 8.23, Found: 49.27; 4.73; 8.30.
¹H NMR (DMSO-*d*₆) δ 2.01 (qui, 2 H), 2.23-2.60 (m, 2 H), 2.71-3.08 (m, 2 H), 3.56 (dd, 1 H), 4.25 (t, 2 H), 7.26 (dd, 1 H), 7.70 (dd, 1 H), 7.85 (dd, 1 H).
¹³C NMR (DMSO-*d*₆) δ 24.37, 25.20, 32.44, 53.03, 69.65, 101.01, 127.92, 128.69, 128.79, 129.37, 161.15, 170.39, 170.97, 175.44.
**MS** (MH+) *m/z*: 341.

### EXAMPLE 8

### [4-[(2-Amino-3 ,4-dioxo-1 -cyclobutene-1 -yl)aminomethyl]-5-(2-thienyl)-3-isoxazolyloxy]acetic Acid, Monohydrate, 8a.

Potassium phthalimide (0.71 g, 3.8 mmol) was suspended in DMF (30 mL) and heated to 90 °C. Ethyl [4-bromomethyl-5-(2-thienyl)-3-isoxazolyloxy]acetate (1.2 g, 3.5 mmol) prepared as previously described in DMF (20 mL) was added over 15 min and the resulting mixture was stirred at 90 °C for 40 min. The mixture was cooled and CH₂Cl₂ (200 mL) and water (200 mL) were added. The phases were separated and the organic phase was washed with saturated CaCl₂ solution (2 x 150 mL), dried (MgSO₄) and concentrated *in vacuo*. Flash chromatography (silica gel, eluent: *n*-heptane/ethyl acetate = 2:1) gave ethyl [4-(*N*-phthalimidomethyl)-5-(2-thienyl)-3-isoxazolyloxy]acetate which was dissolved in CH₂Cl₂ (200 mL), washed with an aqueous saturated CaCl₂ solution (2 x 200 mL), dried (MgSO₄), concentrated *in vacuo* and crystallized from EtOH to give white needle shaped crystals (0.9 g, 64%).
Ethyl [4-(N-phthalimidomethyl)-5-(2-thienyl)-3-isoxazolyloxy]acetate (0.60 g, 1.5 mmol) was boiled under reflux in 1 M NaOH (60 mL) for 45 mm. The solution was cooled, extracted with diethyl ether (3 x 60 mL) and acidified to pH 1-2 with conc. HCl (5 mL). The water phase was extracted with CH₂Cl₂ (3 x 80 mL) and diethyl ether (4 x 80 mL). The combined organic phases were evaporated *in vacuo* to give [4-[*N*-(2-carboxybenzamido)methyl]-5-(2-thienyl)-3-isoxazolyloxy]acetic acid (0.6 g, 100%).
[4-[*N*-(2-carboxybenzamido)methyl]-5-(2-thienyl)-3-isoxazolyloxy]acetic acid (0.60 g, 1.5 mmol) was boiled under reflux in 1 M HCl (125 mL) for 45 min. The solution was cooled, extracted with diethyl ether (4 x 200 mL) and evaporated *in vacuo* to give [4-aminomethyl-5-(2-thienyl)-3-isoxazolyloxy]acetic acid, hydrochloride (0.38 g, 100%).
To a mixture of [4-aminomethyl-5-(2-thienyl)-3-isoxazolyloxy]acetic acid, hydrochloride (0.30 g, 1.0 mmol) and 3-amino-4-ethoxy-3-cyclobutene-1,2-dione (0.16 g, 1.1 mmol) prepared as previously described in EtOH (50 mL) was added NaOH (0.08 g, 2.1 mmol) dissolved in water (3 mL). The mixture was stirred at 22 °C for 18 h. The resulting suspension was evaporated *in vacuo*, dissolved in water and extracted with diethyl ether (2 x 150 mL). The water phase (50 mL) was adjusted to pH 3 with 1 M HCl. The resulting crystals were collected by filtration and dried *in vacuo* to give the title compound, **8a** (0.26 g, 69%). Mp. 222-223 °C (dec.).
CHN; Calcd: 45.78; 3.57; 11.44, Found: 45.13; 3.61; 11.16.
¹H NMR (DMSO-*d*₆) δ 4.75 (d, 2H), 4.86 (s, 2H), 7.30 (dd, 1H), 7.70 (d, 1H), 7.94, (d, 1H).
¹³C NMR (DMSO-*d*₆) δ 34.88, 66.27, 100.97, 127.45, 128.52, 128.67, 130.48, 162.42, 168.19, 168.74, 169.55, 169.95, 183.29.
**MS** (MH+*) m/z*: 350.

### EXAMPLE 9

### (RS)-2-Amino-3-[3-carboxy-5-(2-thienyl)isoxazol-4-yl]propionic acid, hydrate, 9a.

To a cooled solution of sodium (4.1 g, 0.18 mol) in EtOH (100 mL) was added diethyl oxalate (24 mL, 0.18 mol). 1-(2-Thienyl)-1-propanone (20 mL, 0.16 mol) in EtOH (10 mL) was added to the cold solution over 15 min and the resulting mixture was stirred at 0 °C for 2 h followed by 16 h at 22 °C. The mixture was concentrated *in vacuo* and the residue was dissolved in water (400 mL). The aqueous phase was acidified with 1 M HCl to pH 3-4 and extracted with CH₂Cl₂ (3 x 300 mL). The combined organic phases were dried (MgSO₄) and concentrated *in vacuo*. Flash chromatography (silica gel, eluent: *n*-heptane/ethyl acetate = 3:1) gave ethyl 2,4-dioxo-3-methyl-4-(2-thienyl)butyrate (20.5 g, 53%).
To a boiling solution of hydroxylammonium chloride (12.6 g, 0.18 mol) in EtOH (200 mL) was added ethyl 2,4-dioxo-3-methyl-4-(2-thienyl)butyrate in EtOH (60 mL). The resulting mixture was boiled under reflux for 2 h, cooled and concentrated *in vacuo*. Flash chromatography (silica gel, eluent: *n*-heptane/ethyl acetate = 4:1) gave ethyl [4-methyl-5-(2-thienyl)isoxazol-3-yl]carboxylate (13.2 g, 92%).
A mixture of ethyl [4-methyl-5-(2-thienyl)isoxazol-3-yl]carboxylate (8.0 g, 34 mmol), NBS (6.6 g, 37 mmol) and dibenzoylperoxide (0.1 g; 0.4 mmol) in CCl₄ was boiled under reflux for 6 h and left at 22 °C for 14 h. The mixture was cooled, filtered and concentrated *in vacuo* to give ethyl [4-bromomethyl-5-(2-thienyl)isoxazol-3-yl]carboxylate (10.7 g, 100 %).
Ethyl 2-acetylamino-2-ethoxycarbonyl-3-[3-ethoxycarbonyl-5-(2-thienyl)isoxazol-4-yl]proionate was prepared from ethyl [4-bromomethyl-5-(2-thienyl)isoxazol-3-yl]carboxylate in analogy with the method described in Example 7.
A mixture of ethyl 2-acetylamino-2-ethoxycarbonyl-3-[3-ethoxycarbonyl-5-(2-thienyl)isoxazol-4-yl]propionate (1.0 g; 2.2 mmol) and 47 % HBr (aq) (50 mL) was heated to reflux temperature and boiled for 40 min. The resulting solution was evaporated *in vacuo* and water (20 mL) was added. Aqueous NaOH (0.1 M; 22 mL) was added and the mixture was stirred at 22 °C for 2 h. The reaction mixture was evaporated *in vacuo* and water (20 mL) was added. The formed crystals were collected by filtration and dried *in vacuo*. The crystals were dissolved in 47 % HBr and the solution was extracted with diethyl ether which was discharged. The aqueous solution was evaporated *in vacuo* and diethyl ether was added. The reaction mixture was stirred at 22 °C for 16 h and the crystals were collected by decantation of the diethyl ether. The crystals were dried *in vacuo* and aqueous NaOH (0.1 M) was added dropwise to pH 2.5. The formed crystals were collected by filtration and suspended in water (15 mL). The reaction mixture was stirred at 22 °C for 48 h and the resulting crystals were collected by filtration to give the title compound, **9a** (270 mg; 43 %). Mp. 227-28 °C (dec.).
CHN; Calcd: 46.07; 3.69; 9.77, Found: 46.15; 3.68; 9.74.
¹H NMR (D₂O; DSS): δ 2.93-3.06 (m, 1H); 3.15-3.24 (m, 1H); 3.46 (dd, 1H); 7.26 (dd, 1H); 7.66-7.72 (m, 2H).
¹³C NMR (D₂O, pH = 12 (NaOD); Dioxane): δ 29,06; 57,18; 111,38; 129,00; 129,01 (2C); 129,79; 162,28; 163,26; 167,76; 182,65.
**MS** (MH+) *m/z*: 283.

### EXAMPLE 10

### (RS)-2-Amino-3-[3-(5-tetrazolyl)-5-(2-thienyl)isoxazol-4-yl]propionic acid, hydrate, 10a.

To a solution of ethyl [4-methyl-5-(2-thienyl)isoxazol-3-yl]carboxylate (3.7 g; 15.6 mmol) prepared as previously described in THF (20 ml) was added aqueous HCl (6 M, 100 mL). The reaction mixture was boiled under reflux for 6 h. The cooled reaction mixture was extracted with diethyl ether (3 x 100 mL) and the combined organic phases were washed with an aqueous saturated solution of NaCl, dried (MgSO₄) and concentrated *in vacuo*. To the residue was added CH₂Cl₂ and the resulting crystals were collected by filtration. The filtrate was evaporated *in vacuo* and an aqueous saturated solution of NaHCO₃ was added. The aqueous phase was washed with CH₂Cl₂ (2 x 60 mL) and acidified to pH 1-2 with 6 M HCl. The aqueous phase was extracted with diethyl ether (3 x 80 mL). The combined organic phases were dried (MgSO₄) and evaporated *in vacuo*. The combined yields of [4-methyl-5-(2-thienyl)isoxazol-3-yl]carboxylic acid was 1.6 g (48 %).
A mixture of [4-methyl-5-(2-thienyl)isoxazol-3-yl]carboxylic acid (4.0 g; 19.1 mmol), SOCl₂ (40 mL) and DMF (0.1 mL) was refluxed for 60 min. The solution was evaporated *in vacuo*. The residue was dissolved in THF (50 mL) and the organic phase was poured onto an aqueous solution of ammonia (25 %) at 0-5 °C. The reaction mixture was stirred at 22 °C for 1 h and extracted with diethyl ether (4 x 250 mL). The combined organic phases were washed with water and an aqueous saturated solution of NaCl, dried (MgSO₄) and concentrated *in vacuo* to give [4-methyl-5-(2-thienyl)isoxazol-3-yl]carboxamide (3.7 g, 93 %).
A solution of [4-methyl-5-(2-thienyl)isoxazol-3-yl]carboxamide (3.4 g; 16.3 mmol) and POCl₃ (40 mL) was refluxed for 20 min. The reaction mixture was evaporated *in vacuo* and the residue was dissolved in diethyl ether. The organic phase was poured onto an ice/water mixture. The phases were separated and the aqueous phase was extracted with diethyl ether (3 x 100 mL). The combined organic phases were washed with water and an aqueous saturated solution of NaCl, dried (MgSO₄) and concentrated *in vacuo* to give [4-methyl-5-(2-thienyl)isoxazol-3-yl]carbonitrile (2.9 g, 95 %).
[4-Bromomethyl-5-(2-thienyl)isoxazol-3-yl]carbonitrile was prepared from [4-methyl-5-(2-thienyl)isoxazol-3-yl]carbonitrile in analogy with the method described in Example 7.
Ethyl 2-acetylamino-2-ethoxycarbonyl-3-[3-cyano-5-(2-thienyl)isoxazol-4-yl]propionate was prepared from 4-[bromomethyl-5-(2-thienyl)isoxazol-3-yl]carbonitrile in analogy with the method described in Example 7.
A suspension of ethyl 2-acetylamino-2-ethoxycarbonyl-3-[3-cyano-5-(2-thienyl)isoxazol-4-yl]propionate (2.0 g, 4.9 mmol), NaN₃ (0.4 g, 6.2 mmol) and triethylamine hydrochloride (0.9 g, 6.2 mmol) in dimethoxyethane (80 mL) was boiled under reflux for 48 h. Additional NaN₃ (0.4 g, 6.2 mmol) and triethylamine hydrochloride (0.9 g, 6.2 mmol) were added and the reaction mixture was refluxed for further 20 h. The cooled reaction mixture was evaporated *in vacuo* and the residue was subjected to flash chromatography (silica gel, eluent: ethyl acetate/acetic acid = 10:1). Evaporation of the solvent gave ethyl 2-acetylamino-2-ethoxycarbonyl-3-[3-(5-tetrazolyl)-5-(2-thienyl)isoxazol-4-yl]propionate 0.7 g, 32 %).
A mixture of ethyl 2-acetylamino-2-ethoxycarbonyl-3-[3-(5-tetrazolyl)-5-(2-thienyl)isoxazol-4-yl]propionate (0.6 g; 1.3 mmol) and 47 % aqueous HBr (20 mL) was refluxed for 30 min. The reaction mixture was cooled and water (50 mL) was added. The aqueous phase was extracted with diethyl ether (3 x 75 mL). The combined organic phases were extracted with water (50 mL). The combined aqueous phases were evaporated *in vacuo*. Water (25 mL) and aqueous NaOH (0.1 M, 16 mL) was added and the resulting crystals were collected by filtration. The crystals were dried *in vacuo* to give the title compound, **10a** (0.4 g; 90%).
Mp. 209-11 °C (dec.).
¹H NMR (DMSO-*d*₆): δ 3.27-3.54 (m, 2H); 4.37-4.47 (m,1H); 7.33 (dd, 1H); 7.76 (d, 1H); 7.94 (d, 1H).
¹³C NMR (DMSO-*d*₆): δ 24.51; 51.81; 108.03; 127.80; 127.95; 128.57; 129.71; 151.63; 155.91; 161.96; 170.40.
**MS** (MH+) *m/z*: 307.

### EXAMPLE 11

### (RS)-2-Amino-3-[3-hydroxy-5-(2-oxazolyl)isoxazol-4-yl]propionic Acid, Acetate, 11a.

3-Hydroxy-4,5-dimethylisoxazole was prepared by the method as described by Jacquier, R. et al., *Bull. Soc. Chim. Fr.*, **1970**, 2685-90 as modified by Sato, K. et a!., *Agric. Biol. Chem.*, **1986**, *50(7)*, 1831-1837.
A suspension of 3-hydroxy-4,5-dimethylisoxazol (68.5 g, 0.6 mol) and K₂CO₃ (125.6 g, 0.9 mmol) in acetone (1000 mL) was heated to reflux temperature. A solution of ethylbromide (99.1 g, 0.9 mol) in acetone was added dropwise to the boiling reaction mixture. The reaction mixture was stirred for additional 8 h, cooled, filtered and evaporated *in vacuo*. Flash chromatography (silica gel, eluent: ethyl acetate/heptane = 1:3) of the residue gave 3-ethoxy-4,5-dimethylisoxazol (52.7 g, 62 %).
To a cooled (5 °C) mixture of 3-ethoxy-4,5-dimethylisoxazol (65.6 g, 0.5 mol) in CCl₄ (500 mL) was added Br₂ (150 g, 0.9 mol). The reaction mixture was stirred for 96 h at dark place. Water (200 mL) was added and the reaction mixture was decolored by addition of sodiumsulfite. The phases were separated and the aqueous phase was extracted with CH₂Cl₂. The combined organic phases were washed with an aqueous saturated solution of NaCl, dried (MgSO₄) and evaporated *in vacuo*. The residue was dissolved in a water/NMP mixture (15:85, 700 mL) and the reaction mixture was stirred at 100 °C for 24 h. Water was added and the aqueous solution was extracted with diethyl ether. The combined organic phases were washed with water and an aqueous saturated solution of NaCl, dried (MgSO₄) and evaporated *in vacuo*. The residue was subjected to flash chromatography (silica gel, eluent: ethyl acetate/*n*-heptane = 1:2) to give 3-ethoxy-5-hydroxymethyl-4-methylisoxazol (26.3 g; 36 %).
To a solution of 3-ethoxy-5-hydroxymethyl-4-methylisoxazol (25.0 g; 0.16 mol) in a H₂SO₄/H₂O/acetic acid mixture (1:2:7, 200 mL) was added a CrO₃/H₂O/acetic acid mixture (1:1:2, 160 mL). The resulting reaction mixture was stirred for 18 h at 22 °C. Water was added and the aqueous phase was extracted with diethyl ether. The combined organic phases were washed with water and an aqueous saturated solution of NaCl, dried (MgSO₄) and evaporated *in vacuo* to give (3-ethoxy-4-methylisoxazol-5-yl)carboxylic acid (22.7 g; 83 %).
A solution of (3-ethoxy-4-methylisoxazol-5-yl)carboxylic acid (5.0 9; 29 mmol) in CH₂Cl₂ (250 mL), SOCl₂ (4.3 mL) and DMF (0.2 mL) was boiled under reflux for 2 h. The reaction mixture was evaporated *in vacuo*, dissolved in CH₂Cl₂ (100 mL) and added dropwise to a cooled (5 °C) mixture of aminoacetaldehydedimethylacetale (3.5 mL, 3.2 mmol) and K₂CO₃ (6.0 g; 4.4 mmol) in CH₂Cl₂ (100 mL). The resulting solution was stirred for 4 h at 22 °C. Water was added and the phases were separated. The organic phase was washed with water and an aqueous saturated solution of NaCl, dried (MgSO₄) and evaporated *in vacuo* to give 5-[*N*-(acetaldehydedimethylacetale)carboxamide]-3-ethoxy-4-methylisoxazol (7.0 g; 93 %).
A mixture of 5-[*N*-(acetaldehydedimethylacetale)carboxamide]-3-ethoxy-4-methylisoxazol (6.4 g; 25 mmol), P₂O₅ (7.1 g; 50 mmol) and conc. H₂SO₄ (150 mL) was boiled under reflux for 30 min. The cooled (5 °C) reaction mixture was poured onto an ice/water mixture and the aqueous phase was extracted with diethyl ether (2 x 500 mL). The combined organic phases were washed with water and an aqueous saturated solution of NaCl, dried (MgSO₄) and evaporated *in vacuo*. The residue was subjected to flash chromatography (silica gel, eluent: CH₂Cl₂/ethyl acetate/methanol = 1:1:0.5 %) to give 3-hydroxy-4-methyl-5-(2-oxazolyl)isoxazol (340 mg; 8.3 %)
To a cooled (-5 °C) solution of 3-hydroxy-4-methyl-5-(2-oxazolyl)isoxazol (340 mg; 2.0 mmol) in TEA (0.33 mL) and THF (40 mL) was added a solution of benzenesulfonylchloride (0.27 mL, 2.1 mmol) in THF (40 mL). The resulting reaction mixture was stirred at 22 °C for 18 h. Evaporation of the solvent *in vacuo* and flash chromatography (silica gel, eluent: ethyl acetate/*n*-heptane = 1:3) gave 3-benzenesulfonyloxy-4-methyl-5-(2-oxazolyl)isoxazol (520 mg; 85 %).
A mixture of 3-benzenesulfonyloxy-4-methyl-5-(2-oxazolyl)isoxazol (500 mg; 1.6 mmol), NBS (300 mg; 1.7 mmol) and dibenzoylperoxide (0.1 g; 0.4 mmol) in CCl₄ (100 mL) was boiled under reflux for 24 h. The cooled reaction mixture was filtered and evaporated *in vacuo* to give 3-benzenesulfonyloxy-4-bromomethyl-5-(2-oxazolyl)isoxazol (440 mg; 70 %).
Ethyl 2-acetylamino-2-ethoxycarbonyl-3-[3-benzenesulfonyloxy-5-(2-oxazolyl)isoxazol-4-yl]propionate (120 mg; 22%) was prepared from 3-benzenesulfonyloxy-4-bromomethyl-5-(2-oxazolyl)isoxazol (440 mg) in analogy with the method described in Example 7.
To a solution of ethyl 2-acetylamino-2-ethoxycarbonyl-3-[3-benzenesulfonyloxy-5-(2-oxazolyl)isoxazol-4-yl]propionate (120 mg; 0.25 mmol) in methanol (10 mL) was added NaOH (20 mg; 0.5 mmol) in methanol (10 ml). The reaction mixture was stirred for 2 h at 22 °C and the solvent was evaporated *in vacuo*. Water was added to the residue and the aqueous phase was extracted with CH₂Cl₂. The aqueous phase was acidified to pH 2 with 0.1 M HCl and extracted with CH₂Cl₂. The aqueous phase was evaporated *in vacuo* and 1 M aqueous HCl (10 mL) was added. The reaction mixture was boiled under reflux for 3 h. The reaction mixture was evaporated *in vacuo* and ether (10 mL) was added. The resulting crystals were collected by filtration and dried *in vacuo*. The crystals were dissolved in 1 M aqueous HCl and the aqueous phase was washed with diethyl ether. The aqueous phase was evaporated *in vacuo* and the residue was dissolved in water. The aqueous phase was passed through a column containing an ion exchange resin [Amberlite IRA 400, (Cl, 150 mL] using acetic acid (1 M) as an eluent. Evaporation of the solvent gave the title compound, **11a**, (15 mg; 27%).
¹H NMR (D₂O, Dioxane): δ 3.26-3.36 (m, 2H); 4.11-4.21 (m, 1H); 7.40 (s, 1H); 8.04 (s, 1H).
¹³C NMR (D₂O; Dioxane): δ 23.41; 54.17; 105.83; 128.96; 142.16; 152.56; 171.55; 177.26; 177.74.
**MS** (MH+) *m/z*: 240.

### EXAMPLE 12

### (RS)-2-Amino-3-[3-hydroxy-5-(2-thiazolyl)isoxazol-4-yl]propionic Acid, 12a.

A mixture of 5-[*N*-(acetaldehydedimethylacetale)carboxamide]-3-ethoxy-4-methylisoxazol (6.5 g; 25.2 mmol) prepared as described Example 11 and P₂S₅ (5.6 g; 25.2 mmol) in toluene was boiled under reflux for 2 h. The reaction mixture was evaporated *in vacuo* and the residue was subjected to flash chromatography (silica gel, eluent: toluene/ethyl acetate = 11:1) to give 3-ethoxy-4-methyl-5-(2-thiazolyl)isoxazol (0.5 g; 9 %).
A mixture of 3-ethoxy-4-methyl-5-(2-thiazolyl)isoxazol (0.5 g; 2.4 mmol) and NBS (0.5 g; 2.6 mmol) in CCl₄ (50 mL) was boiled under reflux for 36 h. The cooled reaction mixture was filtered and evaporated *in vacuo* to give 4-bromomethyl-3-ethoxy-5-(2-thiazolyl)isoxazol (0.6 g; 87 %).
Ethyl 2-acetylamino-2-ethoxycarbonyl-3-[3-ethoxy-5-(2-thiazolyl)isoxazol-4-yl]propionate (320 mg; 41 %) was prepared from 4-bromomethyl-3-ethoxy-5-(2-thiazolyl)isoxazol (540 mg) in analogy with the method described in Example 7.
A suspension of ethyl 2-acetylamino-2-ethoxycarbonyl-3-[3-ethoxy-5-(2-thiazolyl)isoxazol-4-yl]propionate (245 mg; 0.6 mmol) in 47 % aqueous HBr (5 mL) was boiled under reflux for 30 min. The reaction mixture was evaporated *in vacuo* and water (30 mL) was added. The aqueous solution was treated with charcoal, filtered and evaporated *in vacuo*. The residue was dissolved in water and the aqueous solution was passed through a column containing an ion exchange resin [Amberlite IRA 400, (Cl, 25 mL] using acetic acid (2 M) as an eluent. Evaporation of the solvent gave the title compound, **12a**, (66 mg; 45 %).
¹H NMR (DMSO-*d*₆, 10%, CF₃COOH): δ 3.13-3.27 (m, 1H); 3.30-3.45 (m, 1H); 4.14-4.28 (m, 1H); 8.03 (d, 1H); 8.10 (d, 1H).
¹³C NMR (DMSO-*d*₆, 10%, CF₃COOH): δ 22.98; 51.03; 102.43; 122.64; 144.76; 154.97; 159.81; 170.31; 170.50.
**MS** (MH+) *m/z*: 256.

(*RS*)-2-Amino-3-[3-hydroxy-5-(5-tetrazolyl)isoxazol-4-yl]propionic acid is prepared in a similar manner with the following modifications:

(3-Ethoxy-4-methylisoxazol-5-yl)carboxylic acid prepared as previolusy described is converted to the corresponding carboxamide by conventional methods using SOCl₂ and aqueous ammonia (25 %). The corresponding nitrile compound (3-ethoxy-4-methylisoxazol-5-yl)carbonitrile is obtained by dehydrating the amide with POCl₃. Bromination of (3-ethoxy-4-methylisoxazol-5-yl)carbonitrile with NBS and subsequently reaction with diethyl acetamidomalonate gives ethyl 2-acetylamino-2-ethoxycarbonyl-3-(5-cyano-3-ethoxyisoxazol-4-yl)propionate. Formation of the corresponding 5-(5-tetrazolyl) compound is preferably done by reaction of ethyl 2-acetylamino-2-ethoxycarbonyl-3-(5-cyano-3-ethoxyisoxazol-4-yl)propionate with NaN₃ and triethylamine hydrochloride in dimethoxyethane. Deprotection of ethyl 2-acetylamino-2-ethoxycarbonyl-3-[3-ethoxy-5-(5-tetrazolyl)isoxazol-4-yl]propionate is carried out by the use of aqueous HBr 47 % to give (*RS*)-2-amino-3-[3-hydroxy-5-(5-tetrazolyl)isoxazol-4-yl]propionic Acid.

### EXAMPLE 13

### (RS)-2-Amino-3-[3-hydroxy-5-(2-pyridyl)isoxazol-4-yl)propionic Acid, Hydrate, 13a.

A solution of 5-(2-pyridyl)isoxazol-3-ol (1.14 g, 7.0 mmol) (prepared from methyl 2,3-dibromo-3-(2-pyridyl)propanoate hydrobromide by a modification of the method described by K. Tomita, *Ann. Sankyo Res. Lab.*, **1973**, *25*, 3-5) and NaOH (0.28 g, 7.0 mmol) in water (10 ml) and ethanol (10 ml) was evaporated to dryness and further dried for 2 h at 0.01 mmHg. The residue was suspended in dry DMF (10 ml) and cooled to -10 °C Dimethyl sulfate (0.73 ml, 7.7 mmol) was added dropwise and the mixture was stirred at -10 °C for 1 h and then at 22 °C for 15 h. The resulting solution was evaporated *in vacuo* Water (15 ml) was added to the residue and the mixture was extracted with methylene chloride (3 x 30 ml). The combined organic phases were dried and evaporated *in vacuo*. The residue was subjected to flash chromatography (silica gel, eluent: toluene/ethyl acetate/glacial acetic acid = 1:1:1%) to give 3-methoxy-5-(2-pyridyl)isoxazole (0.73g, 59%)
A solution of 3-methoxy-5-(2-pyridyl)isoxazole (1.53 g, 8.68 mmol) in dry THF (40 ml) was cooled to -78 °C. A solution of *n* -butyl lithium in hexane (9.0 ml, 1.6 M, 14 mmol) was added during 5 min followed by paraformaldehyde (2.48 g, 83 mmol). The mixture was stirred at -78 °C for 15 min and then at 22 °C for 2 h. The reaction mixture was ecaporated *in vacuo* and water (25 ml) and methylene chloride (40 ml) were added and pH was adjusted to 6 with diluted HCl. The phases were separated and the aquous phase was extracted with methylene chloride (2 x 30 ml). The combined organic phases were dried and evaporated *in vacuo*. 4-Hydroxymethyl-3-methoxy-5-(2-pyridyl)isoxazole was isolated by using flash chromatography (silica gel, eluent: toluene/ethyl acetate = 19:1); in a yield of 357 mg (20%).
A mixture of 4-hydroxymethyl-3-methoxy-5-(2-pyridyl)isoxazole (357 mg, 1.73 mmol) and thionyl chloride (10 ml) was boiled under reflux for 2 h. The reaction mixture was evaporated *in vacuo*. An aqueous solution of sodium hydrogencarbonate (5%, 15 ml) was added to the residue and the mixture was extracted with methylene chloride (2 x 25 ml). The combined organic phases were dried and evaporated *in vacuo* to give 4-chloromethyl-3-methoxy-5-(2-pyridyl)isoxazole (388 mg, 100 %).
Sodium hydride (84 mg, 60 % in oil, 2.09 mmol) was added in small portions to a solution of dimethyl acetamidomalonate (361 mg, 1.91 mmol) in dry DMF (4 ml). The mixture was stirred at 22 °C for 45 min. A solution of 4-chloromethyl-3-methoxy-5-(2-pyridyl)isoxazole (390 mg, 1.74 mmol) in dry DMF (4 ml) was added dropwise to the reaction mixture and the resulting reaction mixture was stirred at 22 °C for additional 6 h. The mixture was evaporated *in vacuo* and water (10 ml) was added to the residue. The aqueous reaction mixture was extracted with methylene chloride. The combined organic phases were dried and evaporated *in vacuo*. Flash chromatography of the residue (silica gel, eluent: toluene/ethyl acetate = 19:1) gave methyl 2-acetylamino-2-methoxycarbonyl-3-[3-methoxy-5-(2-pyridyl)isoxazol-4-yl]propionate (500 mg; 76 %).
A mixture of methyl 2-acetylamino-2-methoxycarbonyl-3-[3-methoxy-5-(2-pyridyl)isoxazol-4-yl]propionate (500 mg, 1.3 mmol) and aqueous hydrobromic acid (47%, 20 ml) was boiled under reflux for 1 h. The mixture was evaporated *in vacuo* and water (10 ml) was added to the residue. The aqueuos solution was treated with charcoal and an aqueous solution of sodium carbonate (2 N) was carefully added to the filtrate until pH 3. After 24 h at 5 °C the precipitate was collected, washed with water and dried *in vacuo* to give the title compound, **13a** (174 mg, 54%).
CHN; Calcd: 52.07; 4.57; 16.56, Found: 52.03; 4.50; 16.38.
¹H NMR (200 MHz, DMSO-*d*₆) δ 8.70 (d, 1H); 7.95 (m, 1H); 7.80 (d, 1H); 7.45 (m, 1H); 3.75 (m, 1H); 3.40 (dd, 1H); 3.15 (dd, 1H).
¹³C NMR (200 MHz, DMSO-*d*₆) δ 171.83; 171.53; 162.88; 149.68; 147.64; 137.82; 124.41; 121.17; 104.86; 53.30; 24.24.

The following compound was prepared in a similar manner:

### (RS)-2-Amino-3-[3-hydroxy-5-(4-pyridyl)isoxazol-4-yl]propionic Acid, Hydrate, 13b.

CHN; Calcd: 49.44; 4.90; 15.72, Found: 49.49; 4.88; 15.83.
¹H NMR (200 MHz, DMSO-*d*₆) δ 8.70 (d, 2H); 7.65 (d, 2H); 3.75 (m, 1H); 3.05 (dd, 1H); 2.90 (dd, 1H).
¹³C NMR (200 MHz, DMSO-*d*₆) δ 172.97; 171.20; 161.73; 150.52; 135.30; 121.01; 105.39; 52.70; 24.69.

### EXAMPLE 14

### (S)-(+)-2-Amino-3-(3-hydroxy-5-phenylisoxazol-4-yl)propionic acid, 14a.

The diastereomeric salt of (*RS*)-2-Amino-3-(3-hydroxy-5-phenylisoxazol-4-yl)propionic acid, 0.5 H₂O (11.0 g; 44 mmol) prepared as described by Christensen, I.T., **1989**, *sapra*, and (*R*)-(+)-1-phenylethylamine (5.1 g; 44 mmol) was precipitated from ethanol (300 ml) at 0 °C.

The crystals were dissolved in water and the mixture was acidified to pH 2.5 by use of 0.1 N hydrochloric acid. Crystalline title compound was collected by filtration (320 mg; 6 %). Mp. 251-53 °C, [α]_{D}: +35.3° (c=0.25, 1 N HCl, 20 °C). ee = 99.0%; CHN: Calcd. 58.05; 4.88; 11.29, Found: 58.03; 5.22; 11.33.

(*R*)-(-)-2-Amino-3-(3-hydroxy-5-phenylisoxazol-4-yl)propionic acid, hydrate, **14b**, was prepared in a similar manner using (*S*)-(-)-1-phenylethylamine (5.1 g; 44 mmol). The yield of the title compound, H₂O was 1.4 g; 26 %. The water content was determined to be 7.7 %. Mp. 252-54 °C, [ α]_{D}: -37.8 °(c=1, 1 N HCl, 20 °C). ee = 99.8%; CHN: Calcd. 54.12; 5.31; 10.52, Found: 54.10; 5.27; 10.54.

The following compounds were prepared in a similar manner from (*RS*)-2-Amino-3-[5-(4-fluorophenyl)-3-hydroxyisoxazol-4-yl]propionic acid prepared in analogy with (*RS*)-2-amino-3-[3-hydroxy-5-(2-thienyl)isoxazol-4-yl]propionic acid as described in Example 1:

### (-)-2-Amino-3-[5-(4-fluorophenyl)-3-hydroxyisoxazol-4-yl]propionic Acid, 14c.

Mp. 247 °C (dec.); ee = 99.6 %; CHN; Calcd: 54.12; 4.16; 10.56, Found: 54.16; 4.12; 10.48.
¹H NMR (200 MHz, D₂O, NaOD, Dioxane) δ 2.56 (dd, 1H), 2.82 (dd, 1H), 3.40 (dd, 1H),7.22 (dd, 2H), 7.67 (dd, 2H).

### (+)-2-Amino-3-[5-(4-fluorophenyl)-3-hydroxyisoxazol-4-yl]propionic Acid, 14d.

Mp. 246 °C (dec.); [ α]_{D}: + 37.9° (c=0.25, 1 N HCl, 20 °C); ee = 99.8 %; CHN:
Calcd: 54.12; 4.16; 10.56, Found: 54.12; 4.06; 10.45.
¹H NMR (200 MHz, D₂O, NaOD, Dioxane) δ identical to **14c**.

### EXAMPLE 15

### (-)-2-Amino-3-[3-hydroxy-5-(2-thienyl)isoxazol-4-yl]propionic Acid, 15a.

Compound **1a** (20.8 g; 81.8 mmol) prepared as described in example 1 was added to a mixture of ethanol (350 mL) and acetyl chloride (35 mL) at 25 °C. The resulting reaction mixture was boiled under reflux for 3.5 h. The cooled solution was evaporated *in vacuo* to give (*R S*)-2-amino-3-[3-ethoxy-5-(2-thienyl)isoxazol-4-yl]propionic acid (25.0 g; 96 %).
A solution of (*R S*)-2-amino-3-[3-ethoxy-5-(2-thienyl)isoxazol-4-yl]propionic acid (24.9 g; 78.1 mmol) in CH₂Cl₂ (1200 mL) and TEA (45 mL) was heated to reflux temperature. (*S*)-(+)-2-methoxy-2-phenylacetyl chloride (16.2 g; 87.9 mmol) prepared by conventional methods from (*S*)-(+)-2-methoxy-2-phenylacetic acid in CH₂Cl₂ (250 mL) was added to the hot reaction mixture over 40 min. The resulting reaction mixture was boiled under reflux for further 1 h. The cooled reaction mixture was evaporated *in vacuo* and subjected to flash chromatography (silica gel, eluent: *n*-heptane/ethyl acetate/acetic acid = 55:45:1) to give (*RS*)-ethyl 2-[*N*-(2-methoxy-2-phenylacetamido)]-3-[3-hydroxy-5-(2-thienyl)isoxazol-4-yl]propionate (27.2 g; 81 %).
To a cooled (0°C) solution of (*RS* )-ethyl 2-[*N*-(2-methoxy-2-phenylacetamido)]-3-[3-hydroxy-5-(2-thienyl)isoxazol-4-yl]propionate (27.2 g; 61.1 mmol) in dry THF (600 mL) and TEA (10 mL) was added benzenesulfonyl chloride (12.5 g; 70.8 mmol) in dry THF (300 mL) over 160 min. The resulting solution was stirred at 22 °C for 18 h. The reaction mixture was evaporated *in vacuo* and water and CH₂Cl₂ were added. The phases were separated and and the organic phase was dried (MgSO₄) and evaporated *in vacuo*. The residue containing the two diastereomers of (*RS*)-ethyl 2-[*N*-(2-methoxy-2-phenylacetamido)]-3-[3-benzenesulfonyloxy-5-(2-thienyl)isoxazol-4-yl]propionate was subjected to flash chromatography (silica gel, eluent: toluene/ethyl acetate = 7:1). The yield of the first eluted isomer (Isomer 1) was 10.4 g (60 %) and the yield of the second eluted isomer (Isomer 2) was 9.0 g (51 %).
Isomer 1, (10.4 g; 18.2 mmol) was dissolved in methanol (600 mL). NaOH (0.8 g; 20.0 mmol) in methanol (20 mL) was added and the resulting reaction mixture was stirred at 22 °C for 10 min. Water (5000 mL) was added to the reaction mixture and pH in the aqueous solution was adjusted to 1 with conc. aqueous HCl. The aqueous solution was extracted with diethyl ether and CH₂Cl₂. The combined organic phases were dried and evaporated *in vacuo*. Flash chromatography (silica gel, eluent: ethyl acetate/*n*-heptane/acetic acid = 50:50:1) gave Isomer 1 of (*RS*)-ethyl 2-[*N*-(2-methoxy-2-phenylacetamido)]-3-[3-hydroxy-5-(2-thienyl)isoxazol-4-yl]-propionate (5.8 g; 74 %).
A mixture of the 1-Isomer thus obtained (5.8 g; 13.5 mmol) and aqueous 47 % HBr (250 mL) was boiled under reflux for 1 h. The reaction mixture was evaporated *in vacuo* and water was added. The aqueous solution was washed with CH₂Cl₂ and treated with charcoal. The volume of the aqueous solution was reduced to 200 mL by evaporation *in vacuo*. The zwitterion of Isomer 1 was crystallized from the aqueous solution by addition of aqueous NaOH to pH 3. The resulting crystals were collected by filtration to give (-)-2-amino-3-[3-hydroxy-5-(2-thienyl)isoxazol-4-yl]propionic acid, **15a** (1.0 g; 30%). Mp. 258-60 °C (dec.); [ α]_{D}: -26.8° (c=0.25, 1 N HCl, 20 °C); ee = 96.9 %; CHN; Calcd: 47.23; 3.97; 11.02, Found: 46.88; 3.97; 10.89; **MS** (MH+) *m/z*: 255.2.

In a similar manner treatment of Isomer 2 gave:
(+)-2-Amino-3-[3-hydroxy-5-(2-thienyl)isoxazol-4-yl]propionic acid, **15b**.
Mp. 259-61 °C (dec.); [ α]_{D}: +24.4° (c=0.25, 1 N HCl, 20 °C); ee = 98.9 %; CHN; Calcd: 47.23; 3.97; 11.02, Found: 47.15; 4.05; 10.88; **MS** (MH+) *m/z*: 255.2.

Compound **4c** was treated in a similar way in order to obtain:
(-)-2-Amino-4-[3-hydroxy-5-(2-thienyl)isoxazol-4-yl]butanoic acid, **15c**, Mp. 217-19 °C (dec.); [ α]_{D}: -16.3° (c=0.25, 1 N HCl, 20°C); ee = 85.1 %; and
(+)-2-Amino-4-[3-hydroxy-5-(2-thienyl)isoxazol-4-yl]butanoic acid, **15d**, Mp. 217-19 °C (dec.); [ α]_{D}: +15.6° (c=0.25, 1 N HCl, 20°C); ee = 81.9%.

### Pharmacology

The compounds of the invention were tested in accordance with the following well recognized and reliable test methods. Test results of the receptor binding tests are shown in Table **1**, and the test results of the electophysiological rat cortial wedge preparation are shown in Table **2**.

### [³H]AMPA binding

In this test the affinity of a drug for AMPA receptors is determined by measuring the ability to displace [³H]AMPA from AMPA receptors.

The test was carried out in accordance with a modified version of the method of Honoré, T. and Nielsen, M., *Neurosci.Lett.* **1985**, *54*, 27-32. The test was carried out in the presence of KSCN. This means that only the [³H]AMPA high affinity binding sites were labelled.

The membrane preparations used were obtained in accordance with the method of Ransom, R.W. and Stec, *J.Neurochem.* **1988**, *51*, 830-836.

### [³H]CPP binding

This is a test for determining the affinity of a drug for the NMDA-receptor. In the test the ability of the drug to displace [³H]CPP (4-(3-phosphonopropyl)piperazine-2-carboxylic acid) from the transmitter binding site of the NMDA-receptor is measured.

The test was performed according to the method described by Murphy, D.E., et al. *J.Pharm.Exp.Ther.* **1987**, *240*, 778-784. The membrane preparations used were prepared as described above.

### The Cortical Wedge Model

The Cortical wedge model is a test in which slices of rat brain is examined *in vitro* in order to quantify the effect of ligands at the Glu-receptors and evaluate the pharmacological profile of the ligands (i.e. agonist/antagonist properties). The test was performed as described by Harrison, N.L. and Simmonds, M.A. *Br.J. Pharmacol.* **1985**, *84*, 381-391 as modified according to Wheatley, P.L. *Br.J.Pharmacol.* **1986**, *87*, 159P.

**Table 1**

| Receptor Binding Data | | |
|---|---|---|
| **Compound** | **[**^{**3**}**H]AMPA Binding IC**_{**50**} **values in µM** | **[**^{**3**}**H]CPP Binding IC**_{**50**} **values in µM** |
| **1a** | 0.28 | >100 |
| **1b** | 3.5 | >100 |
| **2a** | 43.0 | 7.5 |
| **3a** | 9.0 | 21.0 |
| **4a** | >100 | 0.76 |
| **4b** | >100 | 2.5 |
| **4c** | >100 | 0.19 |
| **4d** | >100 | 0.7 |
| **5a** | 33.0 | 22.0 |
| **5b** | 38.0 | 6.9 |
| **14a** | 5.5 | >100 |

**Table 2**

| **Electrophysiological Data (rat cortical wedge praparation)** | | | | |
|---|---|---|---|---|
| **Compound** | **Profile** | **EC**_{**50**} **value (µM)** | **IC**_{**50**} **value (µM)** | **Receptor subtype** |
| **1a** | Agonist | 5.8 | | AMPA |
| **1b** | Agonist | 43.0 | | AMPA |
| **1f** | Agonist | 130 | | AMPA |
| **2a** | Part. Ago.# | 300 | | NMDA |
| **2b** | Agonist | 30.0 | | NMDA |
| **3a** | Agonist | 240 | | AMPA |
| **4a** | Antagonist | | 9.0 | NMDA |
| **4b** | Antagonist | | 18.0 | NMDA |
| **4c** | Antagonist | | 2.5 | NMDA |
| **4d** | Antagonist | | 8.6 | NMDA |
| **5a** | Antagonist | | ND | NMDA/AMPA/KAIN |
| **5b** | Antagonist | | 30 | NMDA |
| **5c** | Antagonist | | ND | NMDA/AMPA |
| **13a** | Agonist | 55.0 | | AMPA |
| **13b** | Agonist | 7.4 | | AMPA |
| **14a** | Agonist | 230 | | AMPA |
| **14b** | Antagonist | | 290.*/950* | AMPA/KAIN |
| **14c** | Antagonist | | 337* | AMPA |
| **14d** | Agonist | 170 | | AMPA |
| **15a** | Antagonist | | 300 | AMPA |
| **15b** | Agonist | 2.7 | | AMPA |

| | | | | |
|---|---|---|---|---|
| *) = Kᵢ value; | | | | |
| ND = not determined; | | | | |
| #) Part. Ago. = Partial Agonist | | | | |

In the Pharmacological characterization in the cortical wedge preparation some of the compounds were found to be agonists at the AMPA and NMDA receptors, other compounds were found to be selective NMDA antagonists, and yet other compounds were found to be unselective antagonists at the AMPA and NMDA receptors, respectively. The compounds showed activity in the µM range.

### Results

It appears from Table **1** that some of the compounds of the invention selectively displace [3H]AMPA from AMPA receptors *in vitro* with affinities in low micromolar concentrations. Other compounds selectively displace [³H]CPP from NMDA receptors *in vitro.* Even other compounds of the invention have been found to displace both [³H]AMPA and [³H]CPP from AMPA and NMDA receptors, respectively, *in vitro*.
In the cortical wedge preparation some of the compounds of the invention were found to be agonists or partial agonists whereas others were found to be antagonists. Accordingly the invention comprise compounds having different agonist/antagonist profiles at the glutamic acid receptors with activities in the low micromolar concentrations.

### Formulation Examples

The pharmaceutical formulations of the invention may be prepared by conventional methods in the art.

For example: Tablets may be prepared by mixing the active ingredient with ordinary adjuvants and/or diluents and subsequently compressing the mixture in a conventional tabletting machine. Examples of adjuvants or diluents comprise: corn starch, lactose, talcum, magnesium stearate, gelatine, lactose, gums, and the like. Any other adjuvant or additive colourings, aroma, preservatives etc. may be used provided that they are compatible with the active ingredients.

Solutions for injections may be prepared by solving the active ingredient and possible additives in a part of the vehicle, preferably sterile water, adjusting the solution to the desired volume, sterilization of the solution and filling in suitable ampules or vials. Any suitable additive conventionally used in the art may be added, such as tonicity agents, preservatives, antioxidants, etc.

Typical examples of recipes for the formulations of the invention are as follows:
1) Tablets containing 5 milligrams of Compound **1a**:

| | |
|---|---|
| Compound **1a** | 5.0 mg |
| Lactose | 60 mg |
| Maize starch | 30 mg |
| Hydroxypropylcellulose | 2.4 mg |
| Microcrystalline cellulose | 19.2 mg |
| Croscarmellose Sodium Type A | 2.4 mg |
| Magnesium stearate | 0.84 mg |

2) Tablets containing 1 milligram of Compound **4c**:

| | |
|---|---|
| Compound **4c** | 1.0 mg |
| Lactose | 46.9 mg |
| Maize starch | 23.5 mg |
| Povidone | 1.8 mg |
| Microcrystalline cellulose | 14.4 mg |
| Croscarmellose Sodium Type A | 1.8 mg |
| Magnesium stearate | 0.63 mg |

3) Syrup containing per millilitre:

| | |
|---|---|
| Compound **1a** | 5.0 mg |
| Sorbitol | 500 mg |
| Hydroxypropylcellulose | 15 mg |
| Glycerol | 50 mg |
| Methyl-paraben | 1 mg |
| Propyl-paraben | 0.1 mg |
| Ethanol | 0.005 ml |
| Flavour | 0.05 mg |
| Saccharin natrium | 0.5 mg |
| Water | ad 1 ml |

4) Solution for injection containing per millilitre:

| | |
|---|---|
| Compound **4a** | 0.5 mg |
| Sorbitol | 5.1 mg |
| Acetic acid | 0.08 mg |
| Water for injection | ad 1 ml |

## Claims

1. A (5-arylisoxazol-4-yl)-or (5-arylisothiazol-4-yl)-substituted 2-amino carboxylic acid compounds having general Formula I 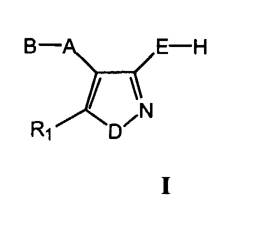
wherein A is a bond or a spacer group selected from C₁₋₆ alkylene, C₂₋₆ alkenylene or C₂₋₆ alkynylene, and cycloalkylene;
E is O, S, COO, (CH₂)ₙ-COO, O-(CH₂)ₙ-COO, or S-(CH₂)ₙ-COO in which groups n is an integer of 1-6, a 5-tetrazolyl group, a 5-tetrazolyl-C₁₋₆ alkyl group, a 3-hydroxyisoxazolyl group or a 3-hydroxyisoxazolyl-C₁₋₆ alkyl group;
D is O or S;
(i) R₁ is an aryl or heteroaryl group optionally substituted with one or more substituents selected from halogen, C₁₋₆ alkyl, C₁₋₆ alkoxy, hydroxy, C₁₋₆ alkylthio, C₁₋₆ alkylsulfonyl, C₁₋₆ alkylamino or di-(C₁₋₆ alkyl)amino, cyano, nitro, trifluoromethyl, or trifluoromethylthio; and
B is a group of Formula II 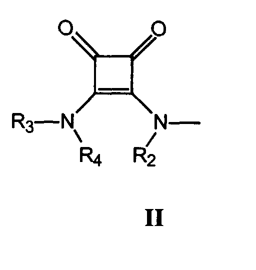
wherein R₂, R₃ and R₄ are independently selected from the group consisting of
a) hydrogen, C₁₋₆ alkyl, C₂₋₆ alkenyl, C₂₋₆ alkynyl, cycloalk(en)yl, cycloalk(en)yl-C₁₋₆ alk(en/yn)yl, phenyl-C₁₋₆ alkyl, thienyl-C₁₋₆ -alkyl, and
b) C₁₋₆ alkyl, C₂₋₆ alkenyl and C₂₋₆ alkynyl in which one or more carbon atoms are replaced by N, O, and/or S; or
R₃ and R₄ are connected thereby forming a C₂-C₆ alkylene, C₂-C₆ alkenylene or C₂-C₆ alkynylene group; or
R₄ and R₂ are connected in order to form a C₁-C₃ alkylene, C₂-C₃ alkenylene or C₂-C₃ alkynylene group optionally mono- or di-substituted with hydroxy or methyl, or CH₂-O-CH₂; or
(ii) R₁ is an aryl group optionally substituted with one or more substituents selected from halogen, C₁₋₆ alkyl, C₁₋₆ alkoxy, hydroxy, C₁₋₆ alkylthio, C₁₋₆ alkylsulfonyl, C₁₋₆ alkylamino or di-(C₁₋₆ alkyl)amino, cyano, nitro, trifluoromethyl, or trifluoromethylthio; and B is a group -CH(NR'R'')-COOH wherein R' and R'' are independently hydrogen or C₁₋₆ alkyl;
provided that when A is methylene, B is a group -CH(NH₂)-COOH, E is O, D is O, and R₁ is phenyl or phenyl substituted with halogen or methoxy; then the compound must be in an enantiomeric pure form.

2. A compound of Claim **1**, **characterised in** that A is a bond or C₁-C₃ alkylene.

3. A compound of Claim **1**, **characterised in** that B is -CH(NR'R'')-COOH or a group of Formula **II** wherein R_{2'} R₃ and R₄ are hydrogen or lower alkyl, or R₄ and R₂ are connected in order to form a C₁-C₃ alkylene group optionally substituted with hydroxy.

4. A compound of Claim **3**, **characterised in** that B is -CH(NH₂)-COOH or a group of Formula **II** wherein each of R_{2'} R₃ and R₄ are hydrogen.

5. A compound of Claim **1**, **characterised in** that E is O, COO,-O-(CH₂)ₙ-COO (n= 1, 2 or 3) or tetrazolyl and D is oxygen.

6. A compound of Claim **1**, **characterised in** that R₁ is an aryl or heteroaryl group selected from the group consisting of 5-membered aromatic heteroaryl groups comprising 1-4 heteroatoms selected from N, O and S such as thienyl, furyl, pyrolyl, oxazolyl, isoxazolyl, thiazolyl, isothiazolyl, pyrazolyl, imidazolyl, triazolyl, oxadiazolyl, thiadiazolyl and tetrazolyl, benzothienyl, benzofuranyl, indolyl, phenyl, biphenyl, pyridyl, pyrimidinyl, pyridazinyl, pyrazinyl, naphthyl, quinolyl, quinazolinyl, quinoxalinyl and cinnolinyl and such aryl or heteroaryl groups substituted with one or more substituents selected from halogen, C₁₋₆ alkyl, C₁₋₆ alkoxy, hydroxy, C₁₋₆ alkylthio, C₁₋₆ alkylsulfonyl, C₁₋₆ alkylamino or di-(C₁₋₆ alkyl)amino, cyano, nitro, trifluoromethyl, or trifluoromethylthio.

7. A compound of Claim **6**, **characterised in** that R₁ is thienyl, oxazolyl, isoxazolyl, thiazolyl, isothiazolyl, pyrazolyl, imidazolyl, oxadiazolyl, thiadiazolyl, tetrazolyl, triazolyl, pyridyl, phenyl, biphenyl or naphthyl or thienyl, oxadiazolyl or phenyl substituted with halogen or methyl.

8. A compound of Claim **7**, **characterised in** that R₁ is 2-thienyl, 3-thienyl, phenyl, 2-pyridyl or 4-pyridyl or 2-thienyl, 3-thienyl or phenyl substituted with halogen or methyl.

9. A compound of Claim **1**, **characterised in** that A is a bond or C₁-C₃ alkylene, B is -CH(NH₂)-COOH or a group of Formula **II** wherein each of R_{3'} R₄ and R₂ are hydrogen, E and D are both oxygen, and R₁ is 2-pyridyl, 4-pyridyl, thienyl, phenyl, substituted thienyl or substituted phenyl.

10. A pharmaceutical compoistion, **characterised in** that it comprises a compound of any of Claims **1 - 9** together with a pharmaceutically acceptable carrier or diluent.

11. Use of a compound of any of Claims **1 - 9** for preparing a pharmaceutical composition for treatment of cerebral ischaemia, Huntington's disease, epileptic disorders, Parkinson's disease, Alzheimer's disease, schizophrenia, pain, depression or anxiety.
